# EUROPÄISCHE PATENTSCHRIFT

(11) **EP 0 995 457 B1**
(45) Veröffentlichungstag und Bekanntmachung des Hinweises auf die Patenterteilung: **10.03.2004**
(21) Anmeldenummer: 99123089.7
(22) Anmeldetag: 05.12.1996
(51) Int. Cl.: A61M 15/00

(54) **Mundstück für einen Inhalator zur mehrfachen dosisweisen Abgabe eines pharmakologischen Trockenpulvers**
Mouthpiece for an inhalator designed to provide multiple doses of dry pharmacological powder
Embout buccal pour un inhalateur fournissant des doses multiples d'une poudre sèche pharmacologique

(30) Priorität: 07.12.1995 CH 346395
(43) Veröffentlichungstag der Anmeldung: 26.04.2000
(62) Teilanmeldung aus: 96938904.8
(73) Patentinhaber: JAGO RESEARCH AG, CH-4132 Muttenz (CH)
(72) Erfinder: Keller, Manfred, 79189 Bad Krozingen (DE); Eggimann, Thomas, 4133 Pratteln (CH)
(74) Vertreter: Heinen, Detlef

(56) Entgegenhaltungen:
- EP-A- 0 129 985
- EP-A- 0 237 507
- WO-A-89/07464
- WO-A-92/05825
- WO-A-93/18811

## Beschreibung

Die Erfindung betrifft ein Mundstück für einen Inhalator zur mehrfachen dosisweisen Abgabe eines pharmakologischen Trockenpulvers gemäss dem Oberbegriff des unabhängigen Patentanspruchs.

Die Inhalation ist eine bewährte Methode, Heilmittel in der Lunge zu deponieren oder dem Blut zuzuführen. Daher wurden zum Zweck der Inhalation, neben den Einrichtungen zum Zerstäuben oder Vernebeln von Flüssigkeiten, z.B. mittels Luft, Kompressoren, Ultraschall, verflüssigten Treibgasen (Fluorkohlenwasserstoffe, Fluor-Chlor-Kohlenwasserstoffe), auch Inhalatoren für pulverförmige Präparate mit dosisweiser Portionierung entwickelt.

Massgeblich bei Inhalatoren ist, dass die Wirkstoffpartikel des Medikaments in einer definierten Dosis und Partikelgrösse (ca. 1-6 µm) durch das Inhalieren entweder in den zentralen oder peripheren Lungenkompartimenten deponiert werden (topische Behandlung) oder als sehr kleine Partikel mittels Absorption im Alveolarbereich in die Blutbahn des Patienten gelangen (systemische Behandlung).

Mikronisierte Partikel mit dem hier relevanten Durchmesser besitzen jedoch äusserst schlechte Fliesseigenschaften. Dieses Problem wird mit verschiedenen konventionellen Verfahren gelöst. So erzeugt man Pulvermischungen mit einem Carrier, der in der Regel einen grösseren Partikeldurchmesser als der Wirkstoff aufweist, wobei sich die Wirkstoffpartikel auf der Carrieroberfläche anlagern. Andererseits werden bei der Herstellung von Softpellets eine Vielzahl von Wirkstoffpartikeln zu jeweils grösseren Partikeln - den Pellets - zusammengeballt. Unter Krafteinwirkung spalten sich die Pellets wieder in die einzelnen, kleineren Wirkstoffpartikel auf. Während der Inhalation sollte es mit dem Inhalator möglich sein, die Wirkstoffpartikel vom Carrier abzulösen bzw. die Pellets wieder in kleine Partikel zu zerlegen. Völlig unerwünscht ist das blosse Verschlucken des Medikaments. Daher bestehen an Inhalatoren grundsätzlich besondere funktionale Anforderungen.

### Stand der Technik

Aus der EP-A-0 404 454 und der EP-A-0 558 879 sind Inhalatoren für den einmaligen Gebrauch bekannt. Derartige Konstruktionen sind nur für spezielle Anwendungen zweckmässig, denn einerseits hat der Patient keine Kontrolle über den ordnungsgemässen Gebrauch, d.h. die optimale Inhalation, und andererseits muss bei jeder Inhalation ein neuer Inhalator benutzt werden, was aufwendig, umständlich und umweltbelastend ist.

Daher wurden auch Inhalatoren mit einem Trockenpulver als Medikament für mehrfachen Gebrauch entwickelt. Aus der WO 93/03782 ist ein Inhalator mit einem Medikamentenreservoir und einem Dosiermechanismus bekannt, mittels dem das Medikament dosisweise vom Vorratsbehälter in den Inhalationskanal gebracht wird und von dort mit dem vom Patienten generierten Luftstrom absaugbar ist. Dieser Inhalator wird noch nicht allen Anforderungen gerecht. Der exakte, vorschriftsgemässe Gebrauch ist noch unzureichend zwingend sichergestellt. Die Dosiergenauigkeit ist zu erhöhen; zu leicht dringt Feuchtigkeit in den Inhalator, die Desagglomeration sowie die Zerstäubung sollten verbessert werden und die Reinhaltung des Inhalators ist kompliziert.

Ein weiterer Inhalator wird in der US-A-5 239 992 offenbart, wo in einer längsverschiebbaren Kolbenstange eine Dosierkavität vorhanden ist, die zunächst unter dem Medikamentenreservoir positioniert, eine Dosis Medikament aufnimmt. Der Patient muss gegen die Kraft einer Feder inhalieren, so dass sich die Kolbenstange verschiebt und die bereitgestellte Dosis über Absaugöffnungen in der Führung der Kolbenstange vom Patienten aufgesogen werden kann. Auch dieser Inhalator weist prinzipiell die vorgenannten Unvollkommenheiten auf.

Mundstücke für bzw. von Inhalatoren sind ebenfalls bekannt, z.B. aus der EP-A-0,237,507. Dort ist das Mundstück integraler Bestandteil des Inhalators. Im Mundstück sind schraubenförmig verlaufende Ablenkwände vorgesehen, um den Luftstrom zu verwirbeln und agglomerierte Partikel wieder zu desagglomerieren.

Ein weiteres Mundstück eines Inhalators ist aus der WO-A-92/05825 bekannt. Es ist ebenfalls integraler Bestandteil des Inhalators. In einer Kammer dieses Mundstücks ist eine Prallplatte angeordnet. Im Luftstrom enthaltene agglomerierte Partikel prallen gegen die Platte, um desagglomeriert zu werden, während inhalative Partikel dem Luftstrom um die Prallplatte herum folgen können.

Ein Mundstück gemäss dem Oberbegriff des unabhängigen Patentanspruchs ist aus der WO-A-93/18811 bekannt. Hier ist ebenfalls eine Prallplatte zur Desagglomeration von agglomerierten Partikeln und zum Umlenken des Luftstroms vorgesehen. Der Kanal, in welchem der Luftstrom zur Prallplatte hinströmt, ist auf einem niedrigeren Niveau angeordnet als der Austrittskanal, sodass gröbere Partikel nur schwer in den Austrittskanal gelangen können.

### Aufgabe der Erfindung

Ausgehend von diesem Stand der Technik liegt der Erfindung die Aufgabe zugrunde, ein gegenüber dem Stand der Technik verbessertes Mundstück vorzuschlagen.

### Übersicht über die Erfindung

Diese Aufgabe wird durch das erfindungsgemässe Mundstück, wie es durch die Merkmale des unabhängigen Patentanspruchs charakterisiert ist, gelöst. Vorteilhafte Ausgestaltungen des erfindungsgemässen Mundstücks ergeben sich aus den Merkmalen der abhängigen Patentansprüche.

### Kurzbeschreibung der beigefügten Zeichnungen

Die folgenden Figuren dienen der Erläuterung des Inhalators insgesamt, betreffen aber nicht das erfindungsgemässe Mundstück:
- Figur 1A: Inhalator, Seitenansicht: geschlossener Zustand (Ausgangslage → Situation A1);
- Figur 1B: Inhalator, Rückansicht;
- Figur 1C: Inhalator, Vorderansicht;
- Figur 1D: Inhalator, Draufsicht;
- Figur 1E: Inhalator: vorgezogene Schutzkappe (Zwischenlage → Situation A2);
- Figur 1F: Inhalator: völlig heruntergeschwenkte Schutzkappe (Inhalations-bereitschaft → Situation A4 / unterlassene Inhalation → Situation A5 / unvollendete Inhalation → Situation A6 / vollendete Inhalation → Situation A7);

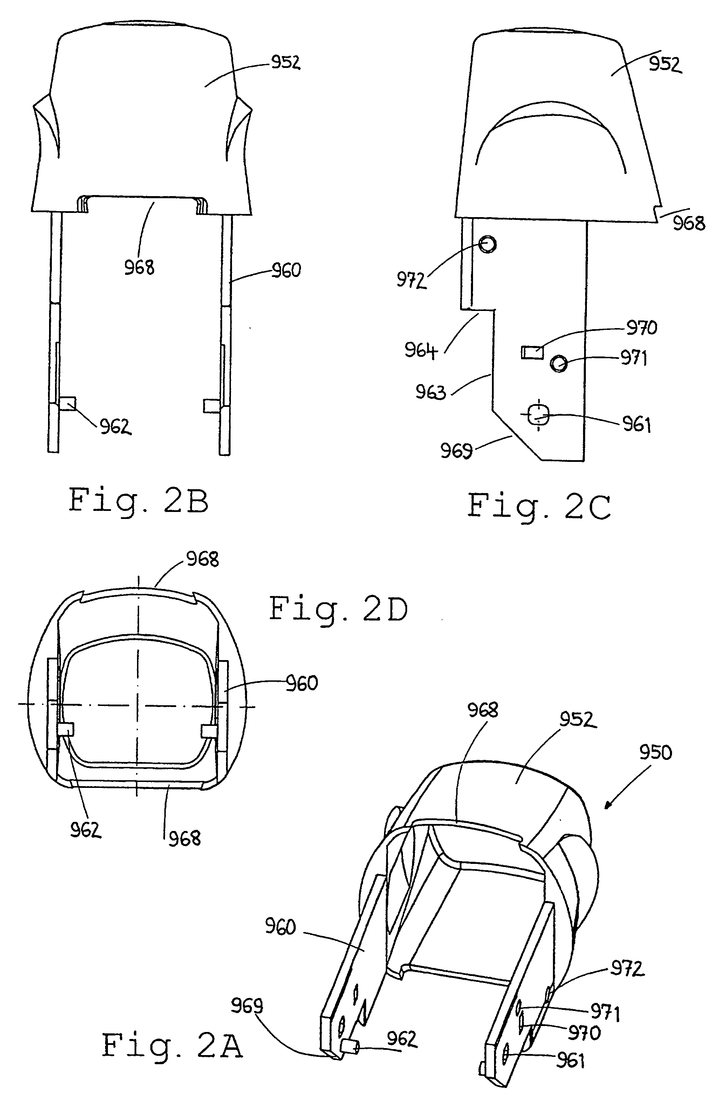

- Figur 2A: Schutzkappe, Perspektivansicht;
- Figur 2B: Schutzkappe, Draufsicht;
- Figur 2C: Schutzkappe, Seitenansicht;
- Figur 2D: Ansicht in die Schutzkappe hinein;

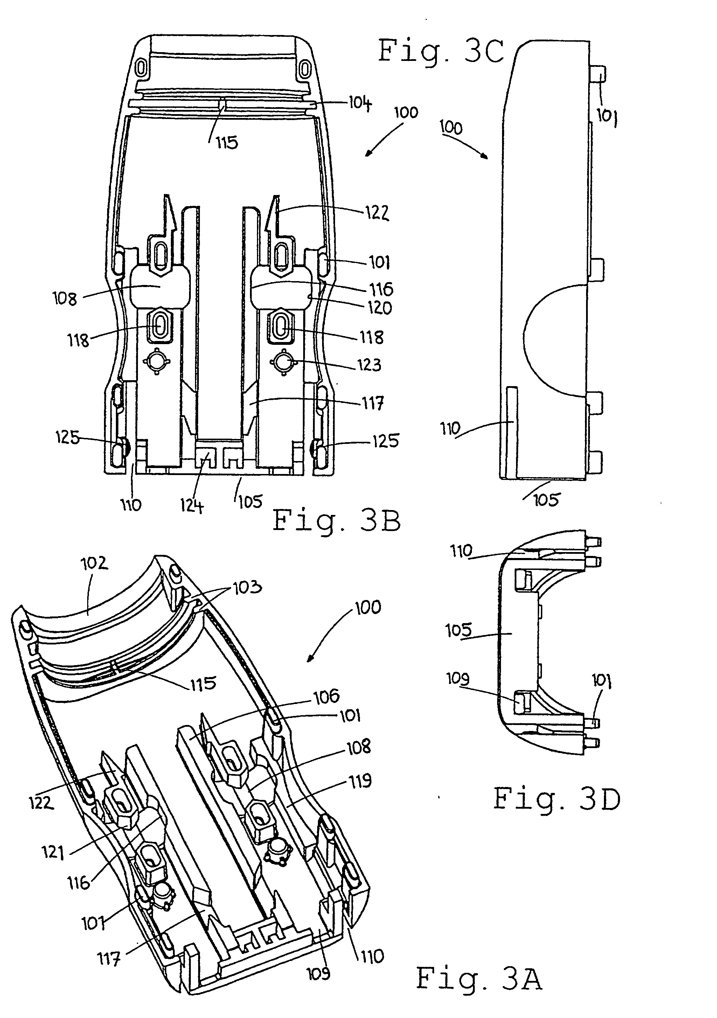

- Figur 3A: Gehäuse-Unterteil, Perspektivansicht;
- Figur 3B: Gehäuse-Unterteil, Draufsicht;
- Figur 3C: Gehäuse-Unterteil, Seitenansicht;
- Figur 3D: Gehäuse-Unterteil, Querschnittsansicht;

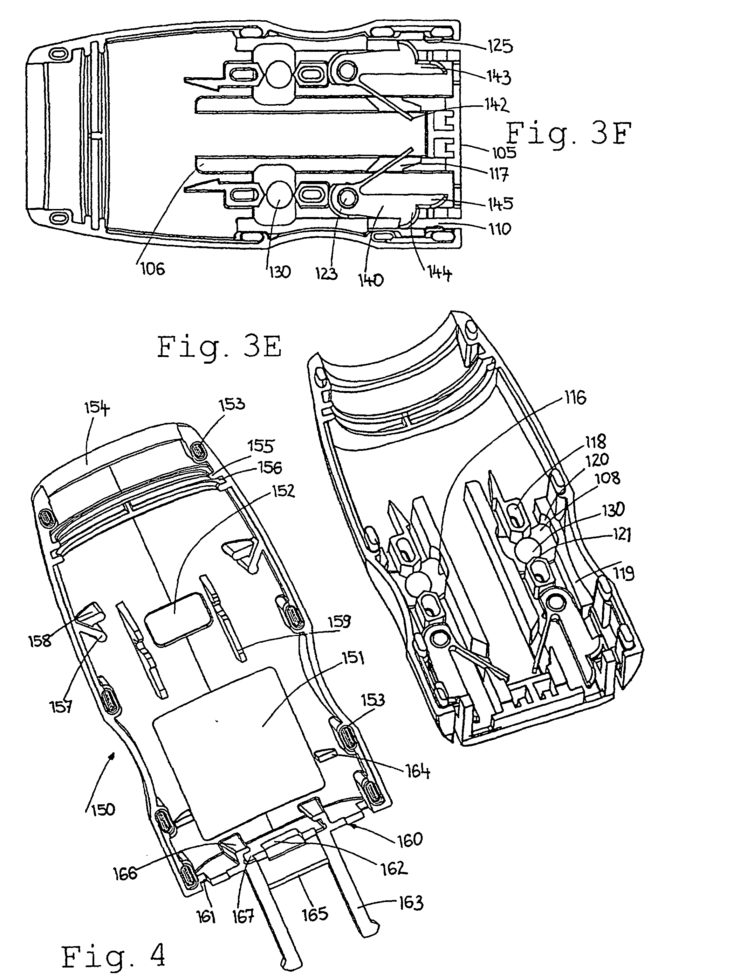

- Figur 3E: Gehäuse-Unterteil, Perspektivansicht mit Blockierhaken und - kugeln;
- Figur 3F: Gehäuse-Unterteil gemäss Figur 3E, Draufsicht;
- Figur 4: Gehäuse-Oberteil, Perspektivansicht;

Die folgenden Figuren dienen ausdrücklich der Erläuterung des erfindungsgemässen Mundstücks:
- Figur 5A: Mundstück, Perspektivansicht auf die Basisplatte;
- Figur 5B: Mundstück, Seitenperspektive;
- Figur 5C: eine Mundstückhälfte, Aussenansicht;
- Figur 5D: eine Mundstückhälfte, Innenperspektive;
- Figur 5E: aufgeklapptes Mundstück, Innenperspektive;

Die folgenden Figuren dienen wieder der Erläuterung des Inhalators insgesamt, betreffen aber nicht das erfindungsgemässe Mundstück:
- Figur 6A: Schieberschiene, Perspektivansicht von unten;
- Figur 6B: Schieberschiene, Seiten perspektive;
- Figur 7A: Schlitten, Perspektivansicht von unten;
- Figur 7B: Schlitten, Perspektivansicht von oben, seitlich, vom;
- Figur 7C: Schlitten, Perspektivansicht von oben, vom;
- Figur 7D: Schlitten, Perspektivansicht von oben, hinten;
- Figur 8A: Dosierschieber, Perspektivansicht von oben;
- Figur 8B: Dosierschieber, Perspektivansicht von oben, hinten;

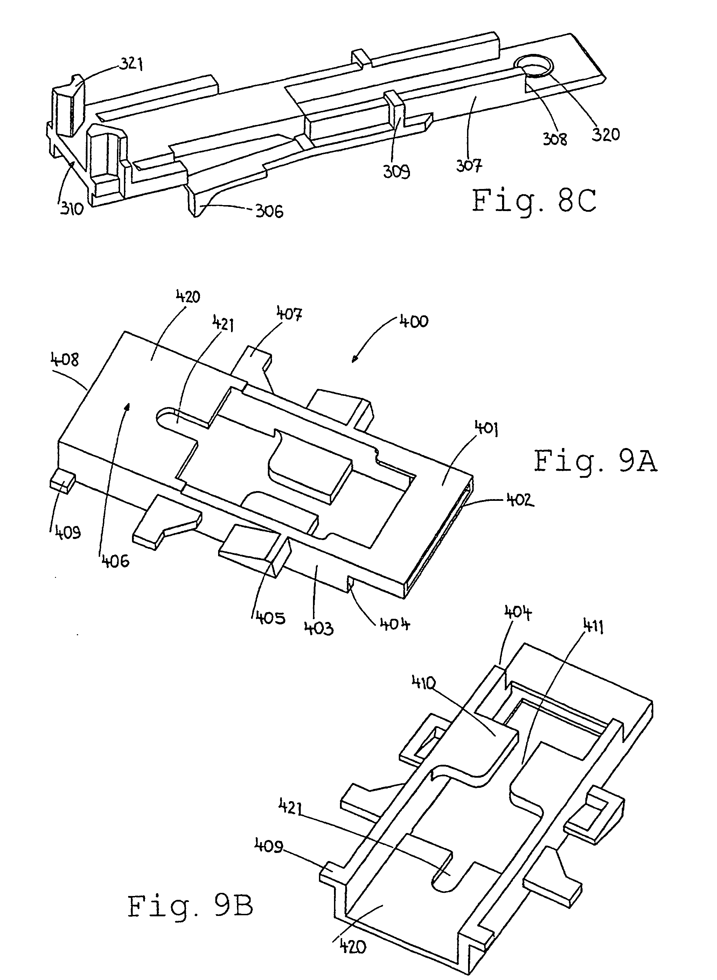

- Figur 8C: Dosierschieber, Perspektivansicht von unten;
- Figur 9A: Zuhalter, Perspektivansicht von oben;
- Figur 9B: Zuhalter, Perspektivansicht von unten;

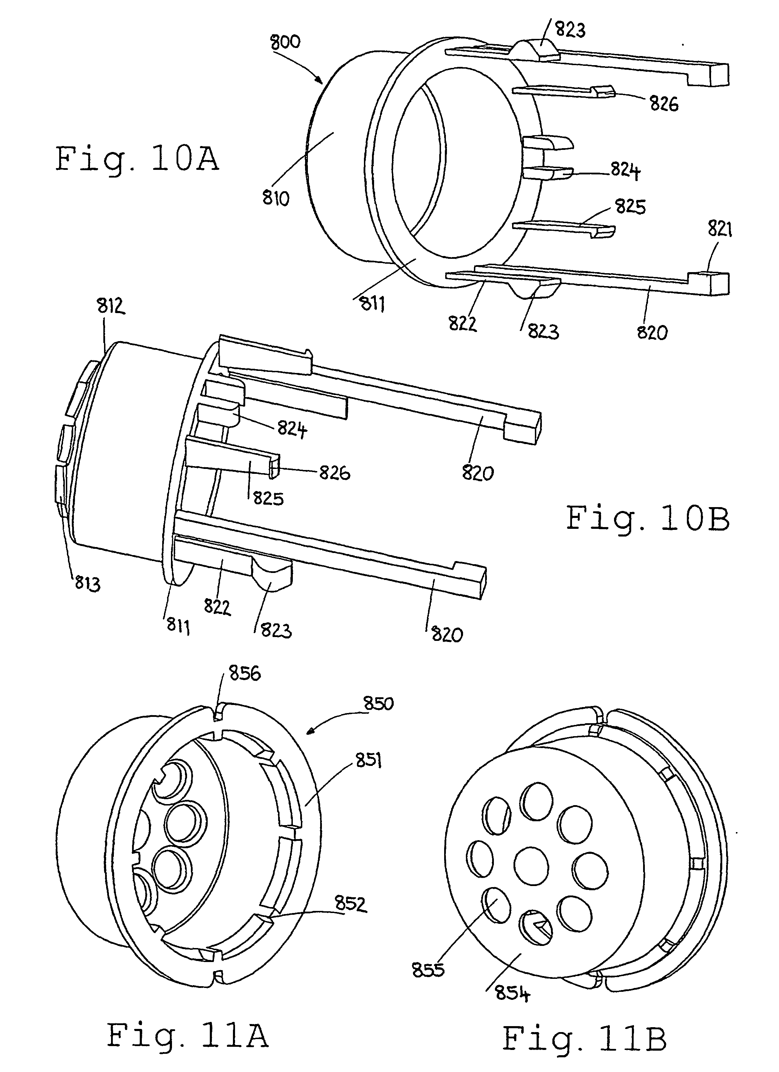

- Figur 10A: Ventilschild, Perspektivansicht;
- Figur 10B: Ventilschild, Seitenperspektive;
- Figur 11A: Ventilführung, Innenperspektive;
- Figur 11B: Ventilführung, Aussenperspektive;
- Figur 12A: Trichter, Perspektivansicht von oben;
- Figur 12B: Trichter, Perspektivansicht von unten;

- Figur 13A: Trichterfassung, Perspektivansicht von oben;
- Figur 13B: Trichterfassung, Seitenperspektive;
- Figur 13C: Trichterfassung, Perspektivansicht von unten;

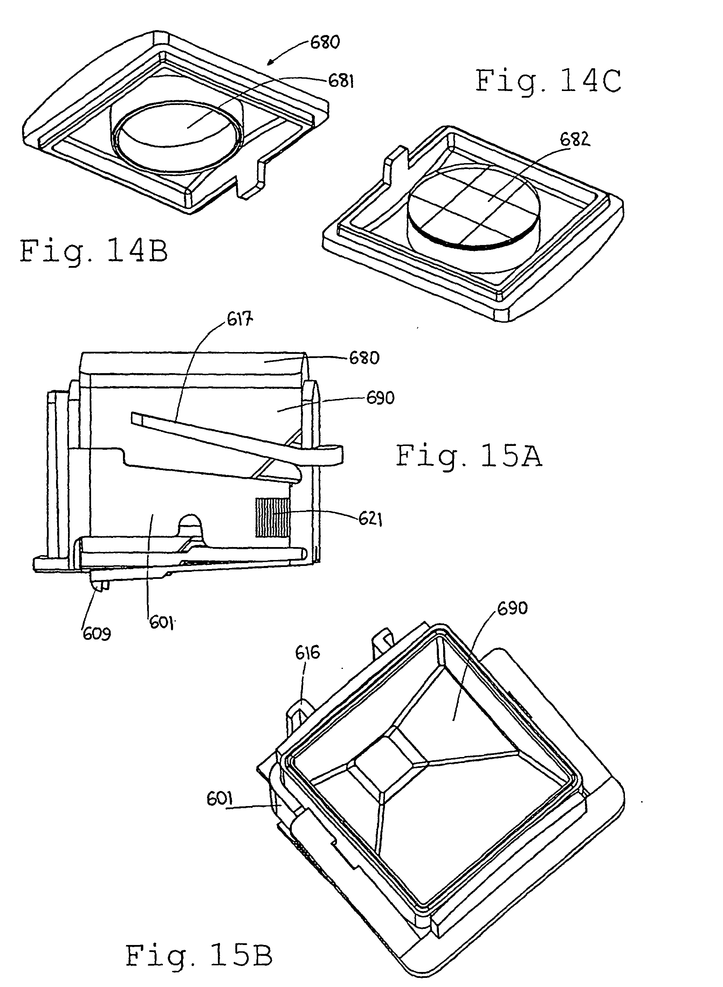

- Figur 14A: Trichterdeckel, Perspektivansicht von oben;
- Figur 14B: Trichterdeckel, Perspektivansicht von unten;
- Figur 14C: Trichterdeckel mit semipermeabler Membran;
- Figur 15A: Trichterfassung, Trichter und Trichterdeckel, Seitenperspektive;
- Figur 15B: Trichterfassung und eingesetzter Trichter, Perspektivansicht von oben;
- Figur 16A: Zähler, Perspektivansicht auf das Einerrad;
- Figur 16B: Zähler, Perspektivansicht auf das Hunderterrad;
- Figur 16C: Zähler-Einerrad, Aussenperspektive;
- Figur 16D: Zähler-Einerrad, Innenperspektive;
- Figur 16E: Zähler-Zehnerrad, Aussenperspektive;
- Figur 16F: Zähler-Zehnerrad, Innenperspektive;
- Figur 16G: Zähler-Hunderterrad, Innenperspektive;
- Figur 16H: Zähler-Hunderterrad, Aussenperspektive;
- Figur 161: Zähler-Stock, Innenperspektive;

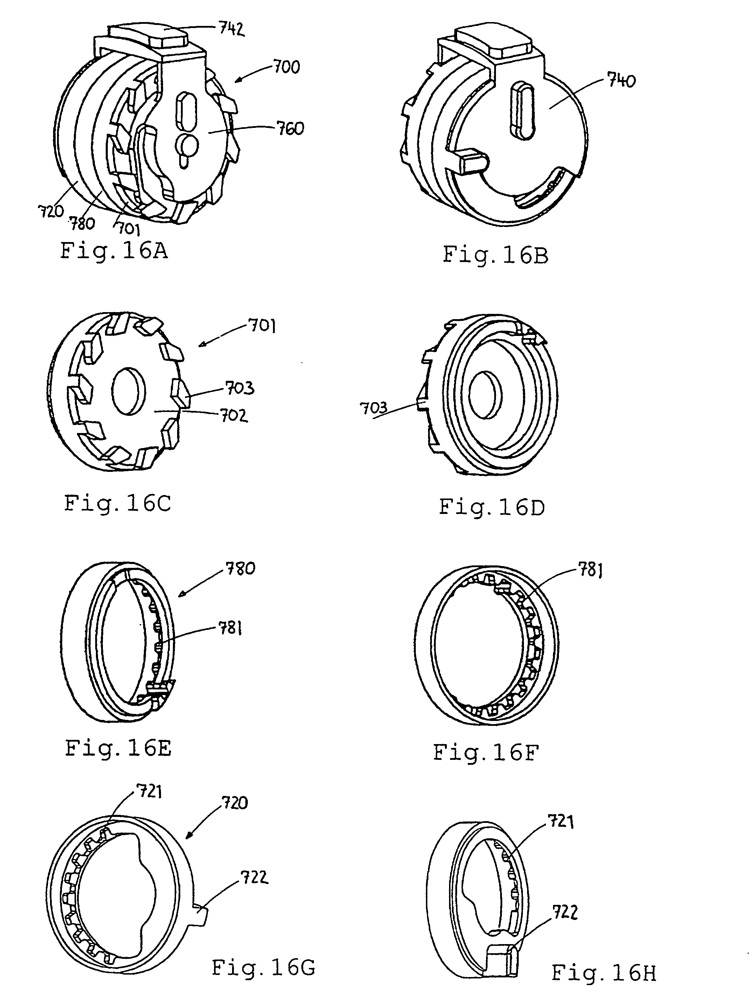

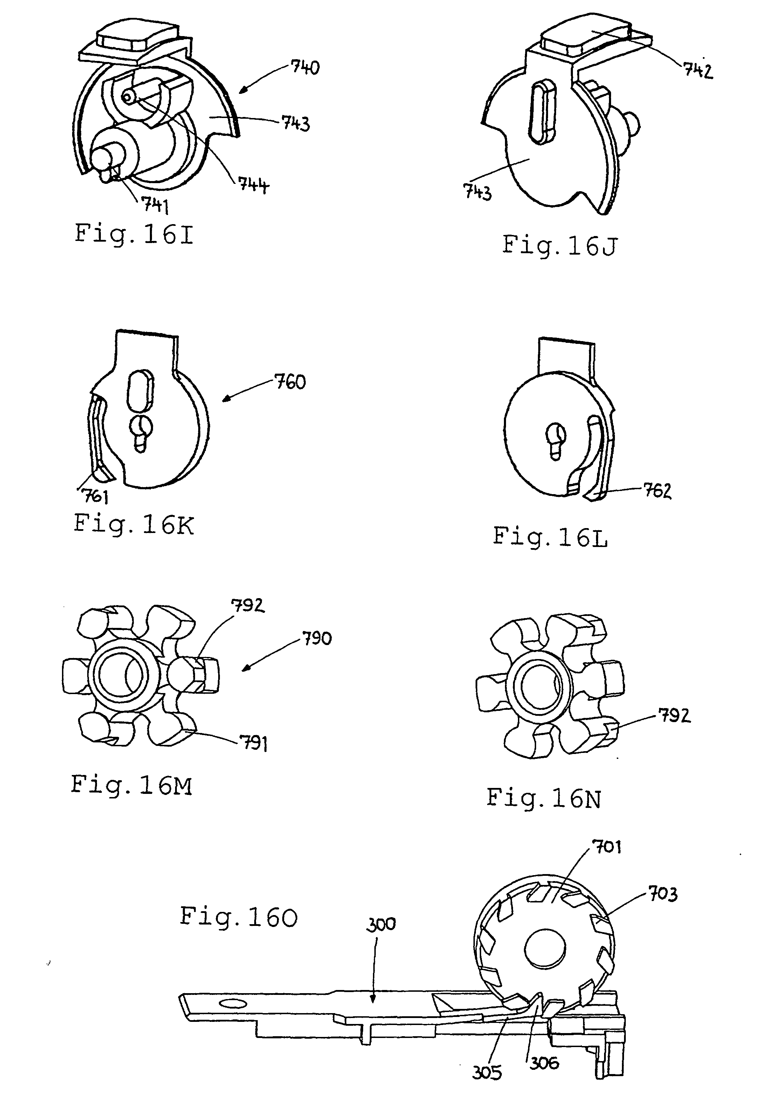

- Figur 16J: Zähler-Stock, Aussenperspektive;
- Figur 16K: Zähler-Deckplatte, Aussenperspektive;
- Figur 16L: Zähler-Deckplatte, Innenperspektive;
- Figur 16M: Zähler-Triebrad, Aussenperspektive;
- Figur 16N: Zähler-Triebrad, Innenperspektive;
- Figur 16O: Angriff des Dosierschiebers am Zähler-Einerrad;

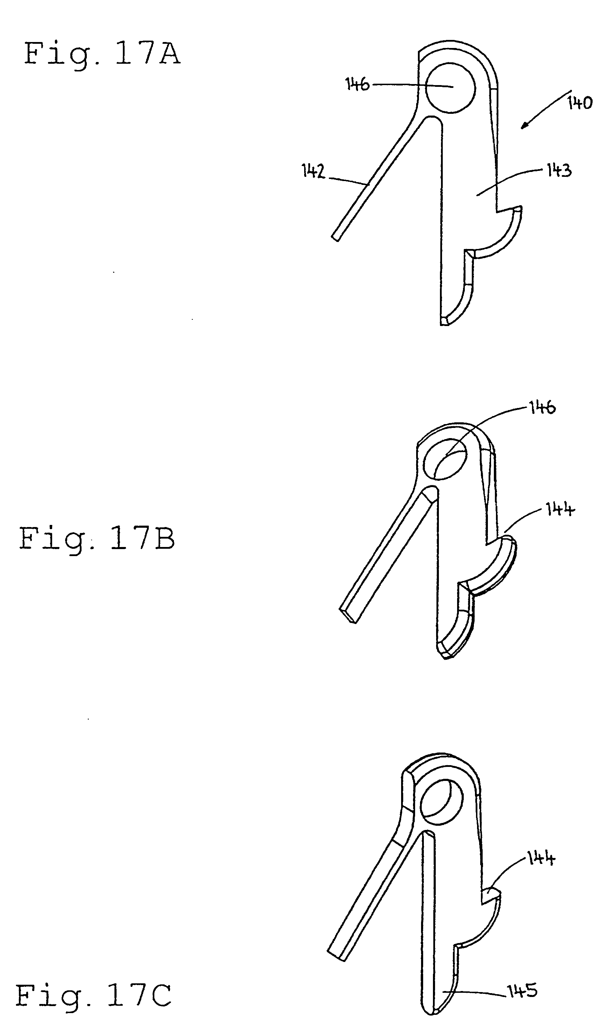

- Figur 17A: Blockierhaken, Draufsicht;
- Figur 17B: Blockierhaken, Perspektivansicht von rechts;
- Figur 17C: Blockierhaken, Perspektivansicht von links;

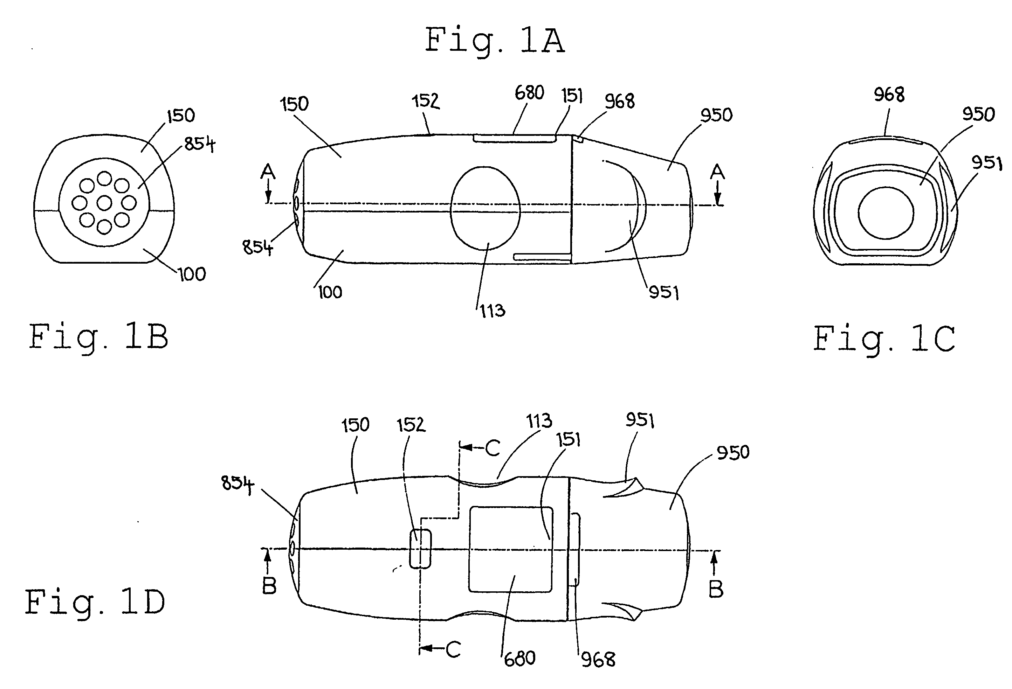

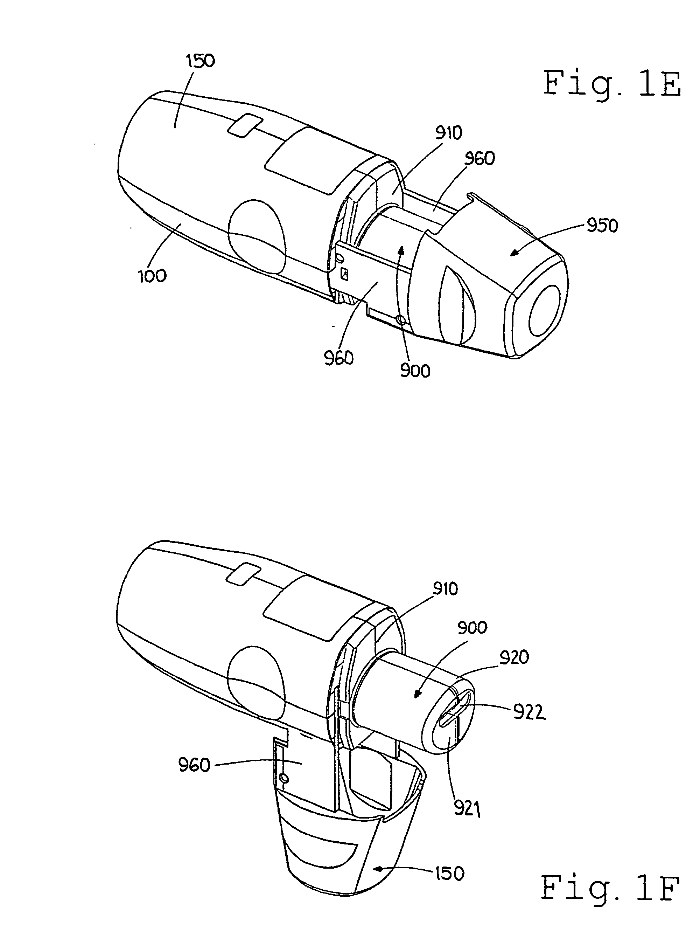

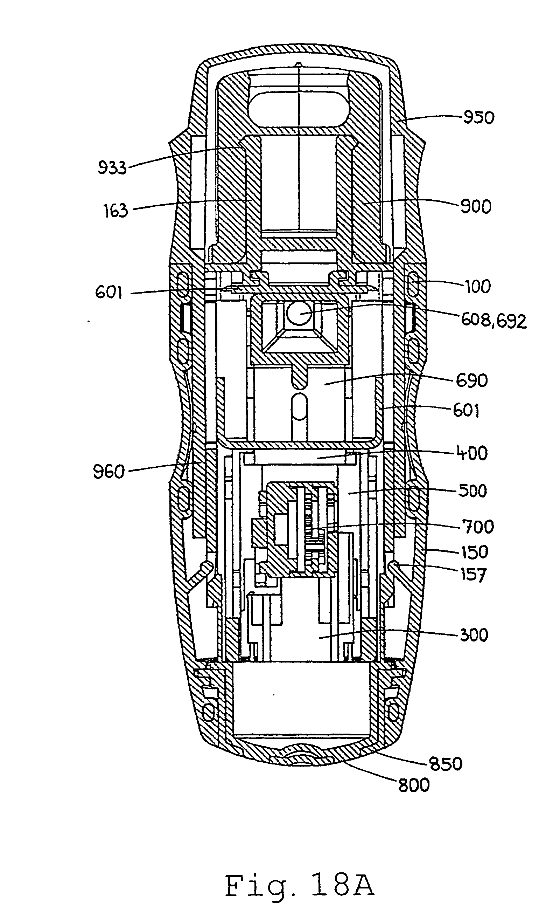

- Figur 18A: Inhalator, horizontaler Längsschnitt gemäss Figur 1A auf der Linie A-A;

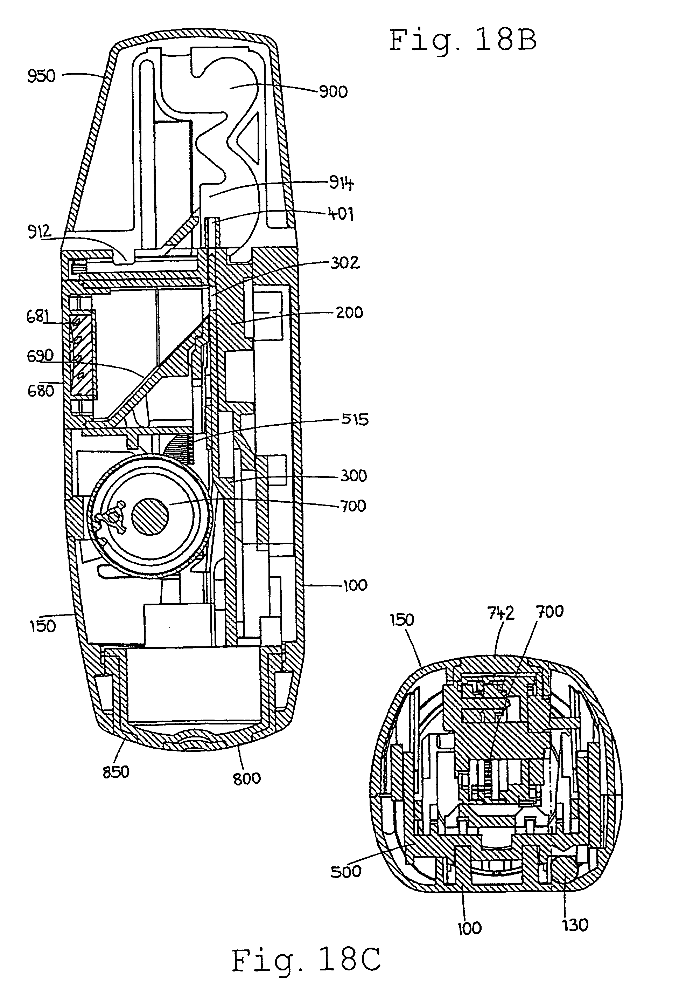

- Figur 18B: Inhalator, vertikaler Längsschnitt gemäss Figur 1D auf der Linie B-B;
- Figur 18C: Inhalator, vertikaler Querschnitt gemäss Figur 1D auf der Linie C-C;

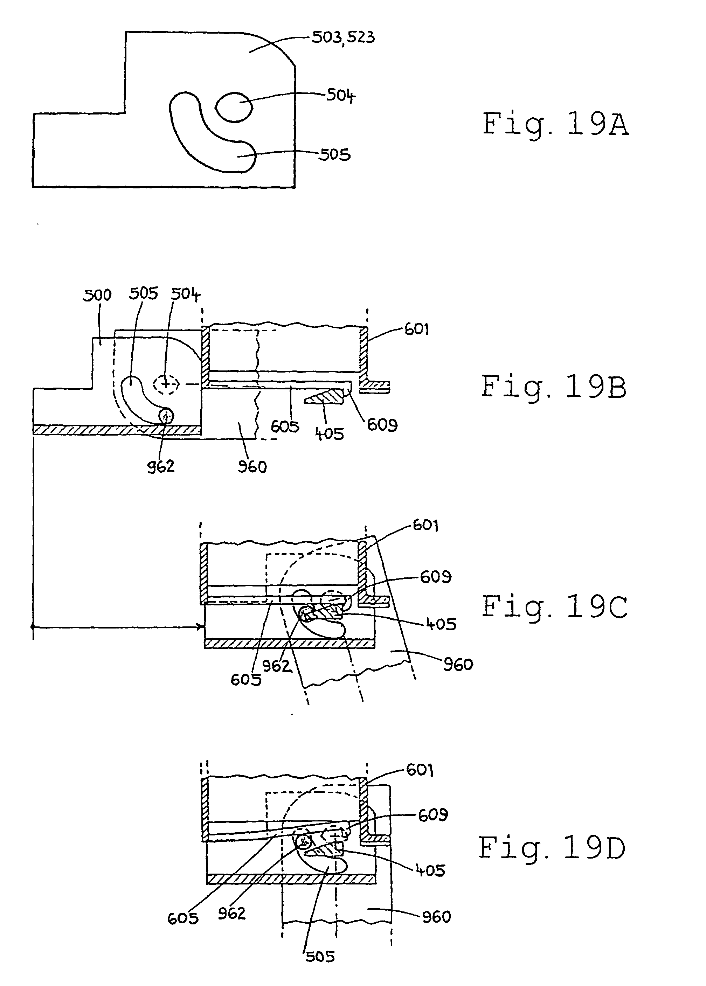

- Figuren 19A bis 19D: Funktionsprinzip der Freigabe des Zuhalters
- Figur 19A: Seitenflügel des Schlittens mit Durchbruch und Nocke
- Figur 19B: geschlossener Inhalator gemäss den Figuren 1A und 18A (*Situation A1*);
- Figur 19C: Zuhalter nahe der Freigabe bei nicht gänzlich heruntergeschwenkter Schutzkappe *(Situation A3);*
- Figur 19D: freigegebener Zuhalter bei gänzlich heruntergeschwenkter Schutzkappe gemäss Figur 1F (*Situation A4*);

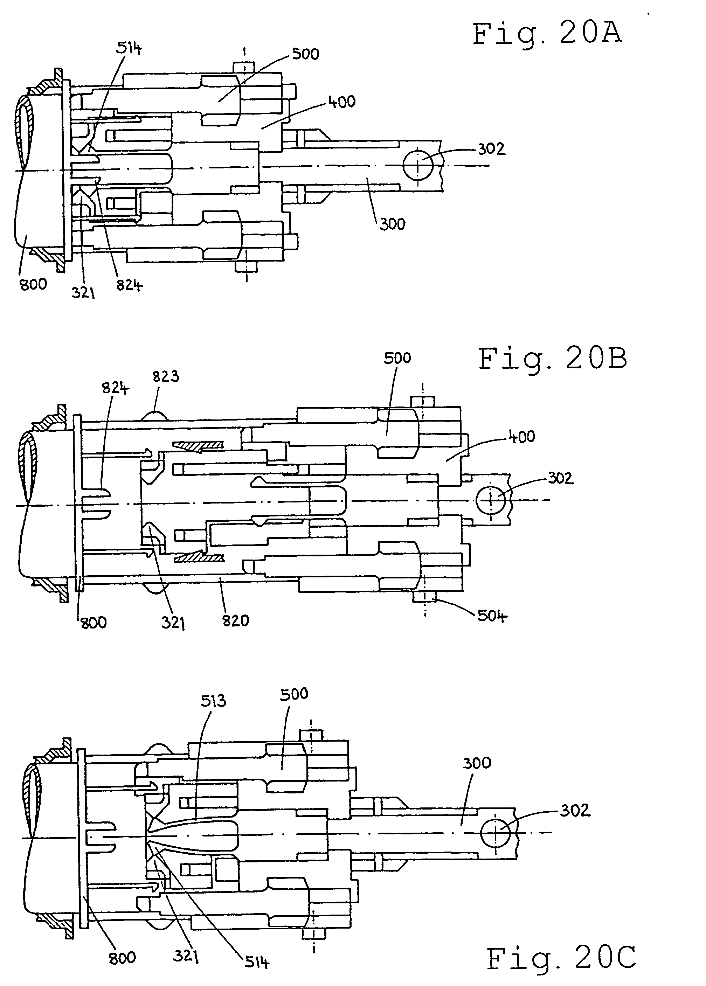

- Figuren 20A bis 20F: Funktionsprinzip des Inhalators
- Figur 20A: geschlossener Inhalator gemäss den Figuren 1A, 18A und 19B (Ausgangslage → *Situation A1*);
- Figur 20B: geöffneter Inhalator gemäss den Figuren 1 F und 19D (Inhalationsbereitschaft → *Situation A4*);
- Figur 20C: Schliessen des Inhalators (unterlassene Inhalation → *Situation A5*);

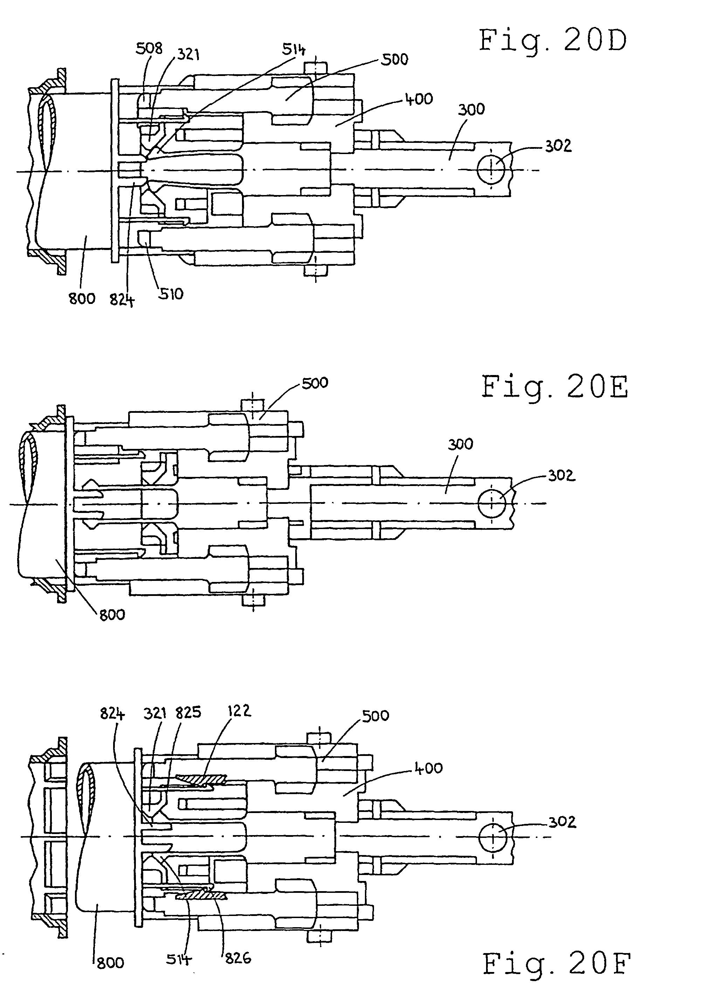

- Figur 20D: Schliessen des Inhalators (unvollendete Inhalation → *Situation* A6);
- Figur 20E: geschlossener Inhalator (nach unvollendeter Inhalation → *Situation A8*);
- Figur 20F: geschlossener Inhalator (nach vollendeter Inhalation → *Situation A7*);

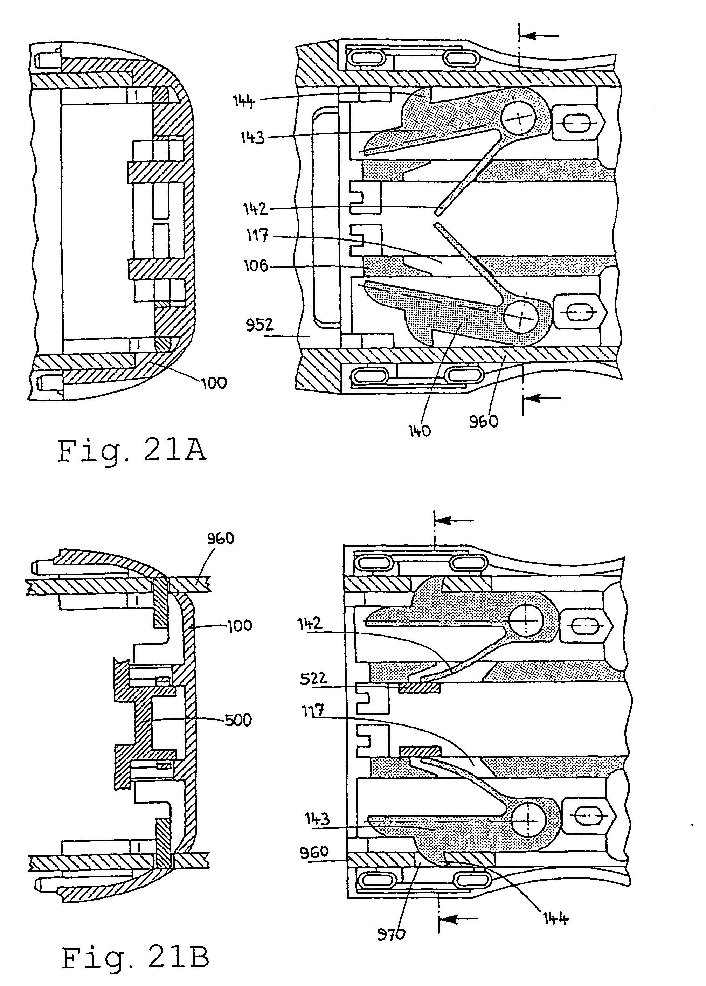

- Figuren 21A bis 21C: Funktionsprinzip der Blockierhaken:
- Figur 21A: eingesetzte Blockierhaken (Ausgangsposition → *Situation B1*);
- Figur 21B: blockierte Schutzkappe bei fehlendem Mundstück (Fehlposition *→ Situation B2*);

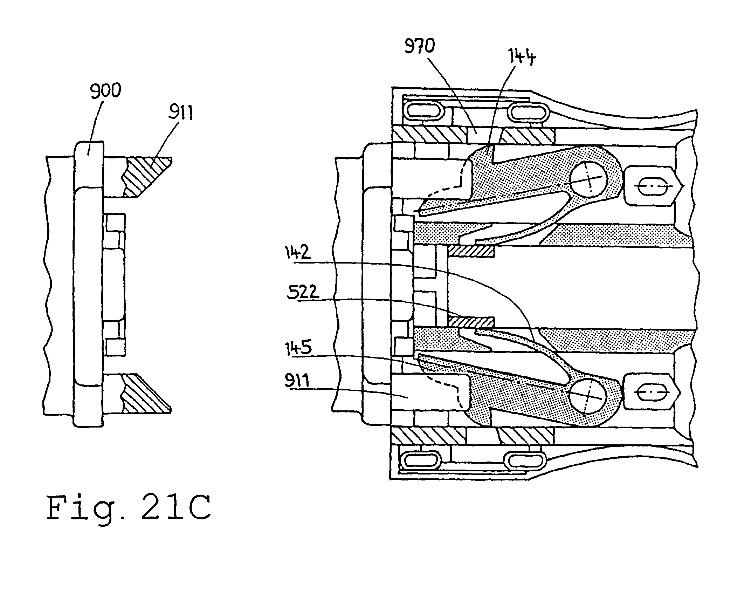

- Figur 21C: schwenkbare Schutzkappe bei eingesetztem Mundstück (Sollposition → *Situation B3*);

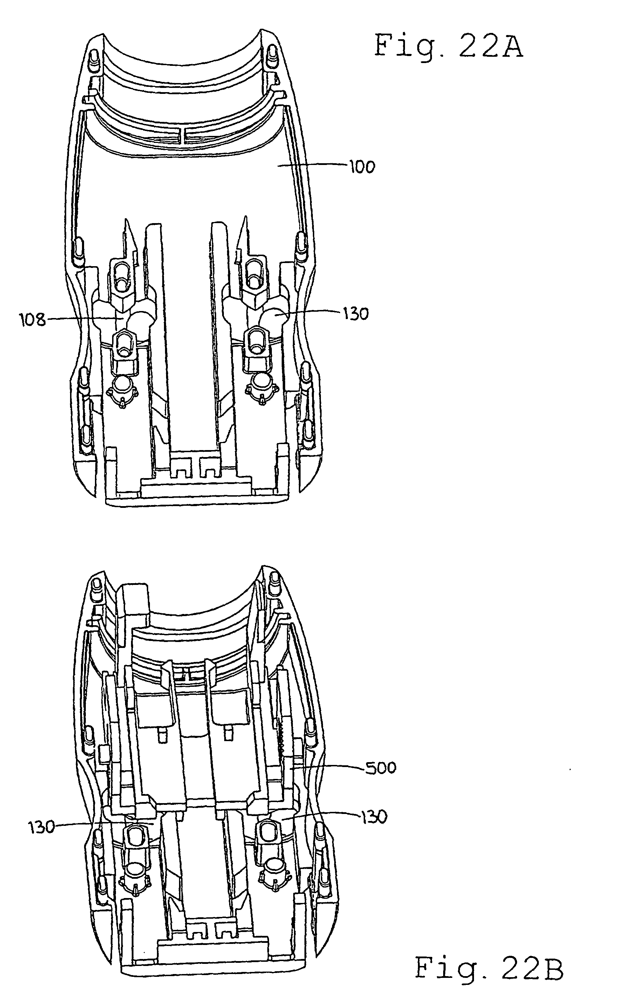

- Figuren 22A und 22B: Funktionsprinzip der Blockierung des Inhalators bei Schräglage:
- Figur 22A: Seitenlage der Blockierkugeln bei Fehlposition des Inhalators;
- Figur 22B: blockierter Inhalator;

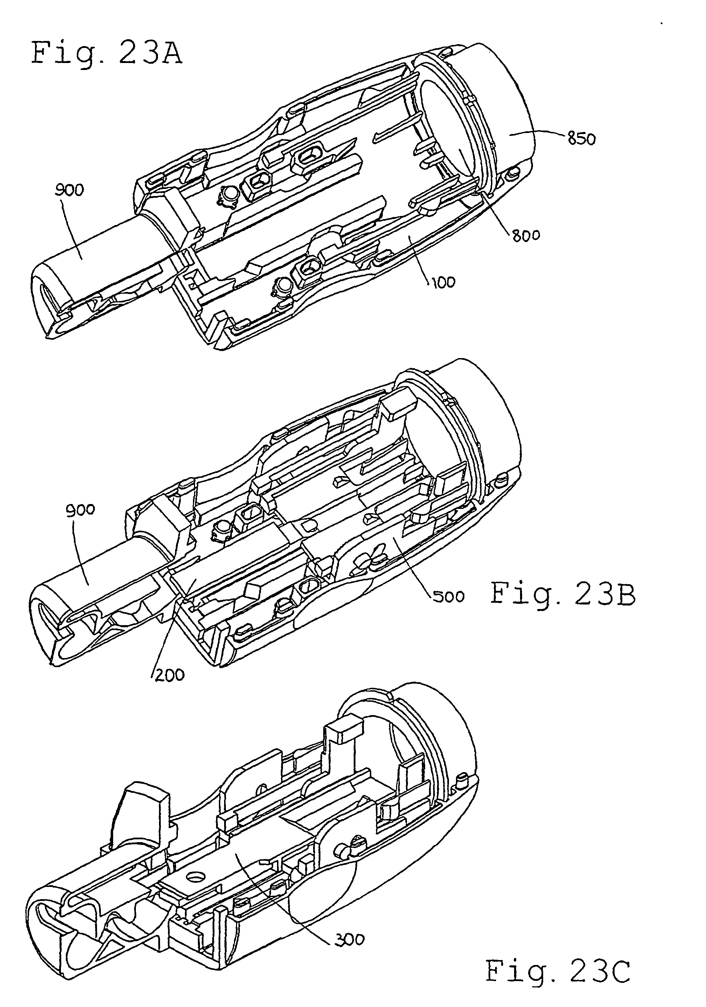

- Figuren 23A bis 23G: sukzessive aufgebauter Inhalator, Perspektivansichten;
- Figur 23A: Gehäuse-Unterteil mit Ventilschild, Ventilführung und einer Mundstückhälfte;
- Figur 23B: Darstellung gemäss Figur 23A ergänzt mit Schieberschiene und Schlitten;
- Figur 23C: Darstellung gemäss Figur 23B ergänzt mit Dosierschieber;

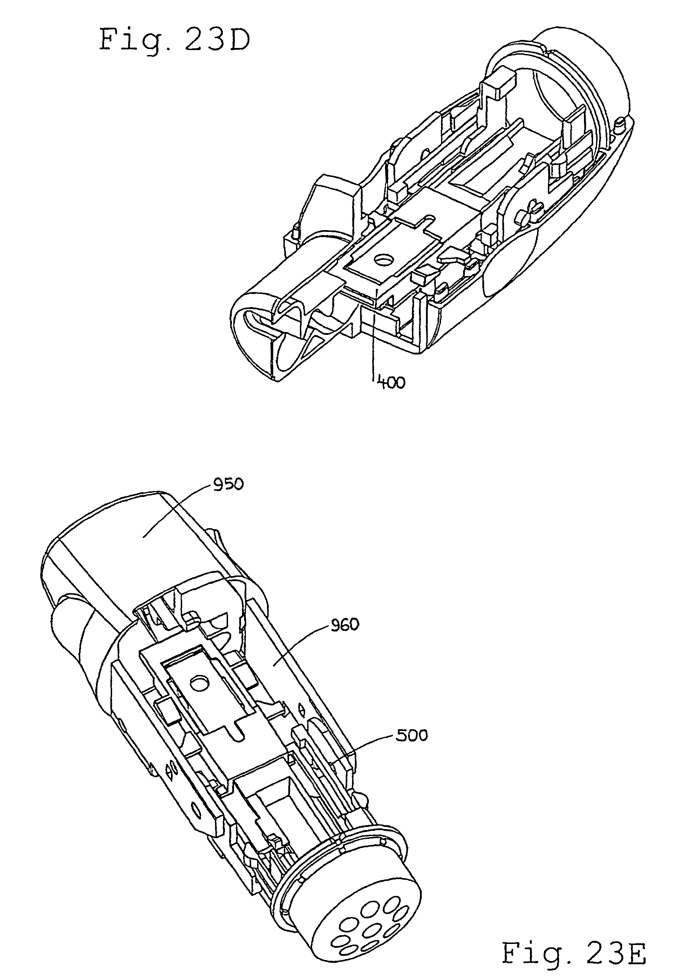

- Figur 23D: Darstellung gemäss Figur 23C ergänzt mit Zuhalter;
- Figur 23E: Darstellung gemäss Figur 23D ergänzt mit Schutzkappe, ohne Gehäuse-Unterteil;

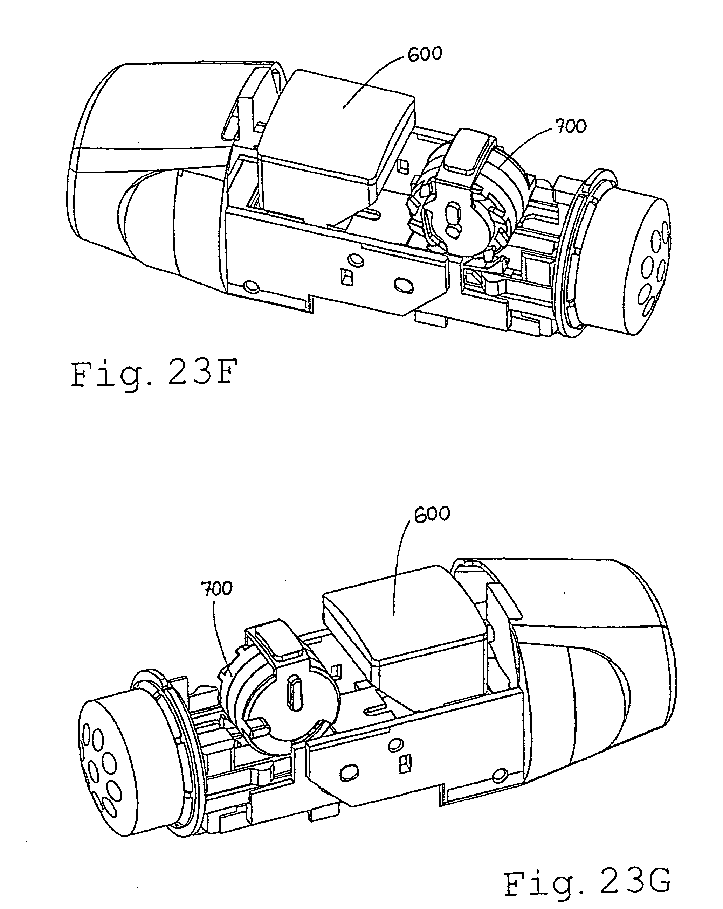

- Figur 23F: Darstellung gemäss Figur 23E ergänzt mit Trichterfassung, Trichter, Trichterdeckel und Zähler; und
- Figur 23G: Darstellung gemäss Figur 23F von Rückseite.

### Ausführungsbeispiel

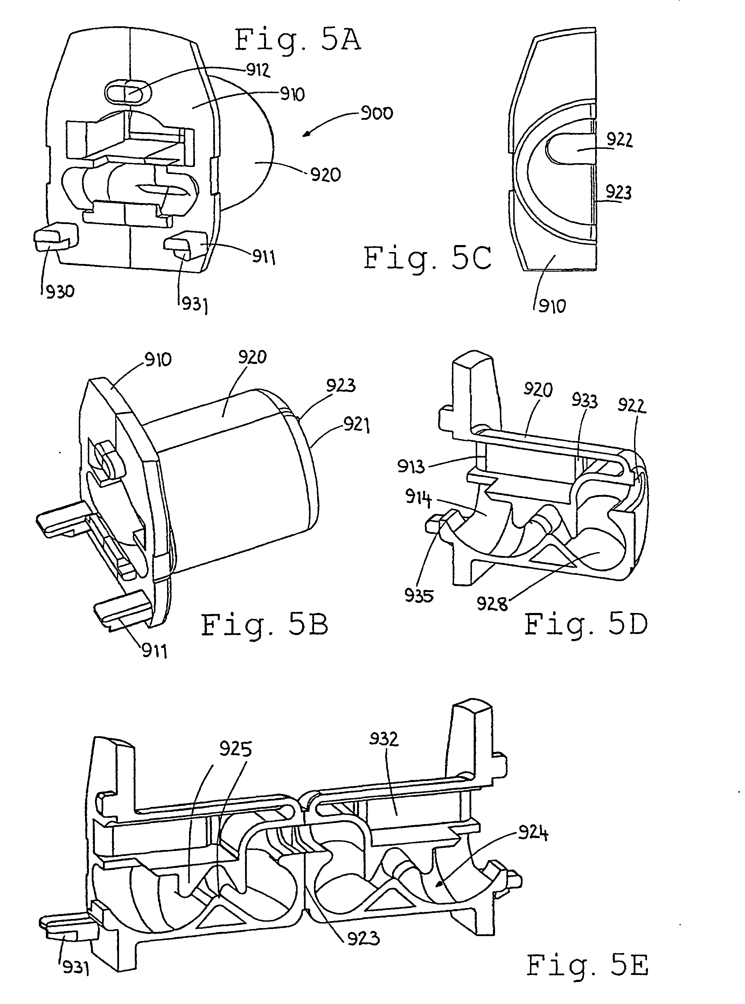

Im folgenden wird der Inhalator, der nicht Gegenstand der Erfindung ist, in seinem Aufbau und seine Funktion unter Bezugnahme auf die beigefügten Zeichnungen detaillierter beschrieben, wobei abschliessend mögliche Modifikationen erwähnt sind. Das erfindungsgemässe Mundstück wird anhand der Figuren 5A-5E erläutert.

Für die gesamte weitere Beschreibung gilt folgende Festlegung. Sind in einer Figur zum Zweck zeichnerischer Eindeutigkeit Bezugsziffem enthalten, aber im unmittelbar zugehörigen Beschreibungstext nicht erläutert, so wird auf deren Erwähnung in vorangehenden Figurenbeschreibungen Bezug genommen. Im Interesse der Übersichtlichkeit wird auf die wiederholte Bezeichnung von Bauteilen in nachfolgenden Figuren zumeist verzichtet, sofern zeichnerisch eindeutig erkennbar ist, dass es sich um ''wiederkehrende'' Bauteile handelt.

### Figuren 1A bis 1D

Äusserlich setzt sich der Inhalator aus dem Gehäuse-Unterteil **100,** dem Gehäuse-Oberteil **150** sowie der Schutzkappe **950** zusammen. Das Gehäuse-Unterteil **100** und das Gehäuse-Oberteil **150** haben eine längliche, halbschalenartige Form. Letztere besitzt an ihrer Oberseite eine grössere Aussparung **151** zur Aufnahme eines Trichterdeckels **680** sowie ein Fenster **152**, durch welches der Stand des Zählers ablesbar ist. Das Gehäuse-Unterteil **100** und das Gehäuse-Oberteil **150** sind aneinandergefügt, so dass sich ein im Prinzip geschlossenes Gehäuse ergibt. Zum besseren Ergreifen sind aussen an der Schutzkappe **950** Griffkonturen **951** vorgesehen. Am Gehäuse-hier sich über das Gehäuse-Unterteil **100** und das Gehäuse-Oberteil **150** erstreckend - sind ebenfalls beiderseits Griffkonturen, bevorzugt als Griffmulden **113** ausgebildet, angeordnet.

Oben in der Schutzkappe **950** ist zum Aussenrand hin eine längliche Aussparung vorgesehen, wodurch mit dem angrenzenden Gehäuse-Oberteil **150** eine Sichtfuge **968** entsteht. Mit einem Blick in diese Sichtfuge **968** lässt sich feststellen, ob das Mundstück eingesetzt und die Sichtfuge **968** damit ausgefüllt ist, oder ob das Mundstück fehlt und die Sichtfuge **968** folglich offen ist. Der Schutzkappe **950** gegenüberliegend - an der Hinterpartie des Inhalators - wird der perforierte Boden **854** der vom Gehäuse-Unterteil **100** und Gehäuse-Oberteil **150** umschlossenen Ventilführung sichtbar.

Im hier gezeigten geschlossenen Zustand, der Ausgangslage - ferner als *Situation A1* bezeichnet -, ist die Schutzkappe **950** bündig bis an das Gehäuse-Unterteil **100** und das Gehäuse-Oberteil **150** heran aufgesteckt. Somit ist das im Inhalator bevorratete medizinische Präparat quasi hermetisch von äusserer Feuchtigkeit geschützt.

### Figur 1E

Vor dem Gebrauch muss der Inhalator geöffnet werden; dazu wird zunächst die Schutzkappe **950** in axialer Richtung abgezogen. Der Auszugsweg der Schutzkappe **950** wird durch ein Paar von an der Schutzkappe **950** fest angeordneten Bügeln **960** begrenzt, welche längsverschiebbar in das Innere des Inhalators eingreifen. Bei soweit abgezogener Schutzkappe **950** wird das Mundstück **900** bereits partiell sichtbar, welches an das Gehäuse-Unterteil **100** und das Gehäuse-Oberteil **150** frontseitig angesetzt ist und von den Bügeln **960** beidseitig umgriffen wird. Wie später dargelegt, ist mit diesem Schritt eine zeitweilige Vibration zur exakten Dosierung des Medikaments aus dem Pulverreservoir verknüpft. Diese Zwischenlage mit vorgezogener Schutzkappe 950 wird im weiteren als *Situation A2* bezeichnet.

### Figur 1F

Um das Mundstück **900** für den Patienten zugänglich zu machen, d.h. die Inhalation zu ermöglichen, muss in einer weiteren Aktion die an den Bügeln **960** hängende Schutzkappe **950** heruntergeschwenkt werden. Nunmehr ist das Mundstück **900** mit dem von der Basisplatte **910** vorstehenden Mundrohr **920** völlig sichtbar. An der Stirnseite **921** des Mundrohrs **920** befindet sich der Kanalausgang **922**, durch welchen der Patient das Medikament inhaliert.

Bei dieser Lage mit vorgezogener und völlig heruntergeschwenkter Schutzkappe - ferner als *Situation A4* bezeichnet - ist der Inhalator an sich zur Inhalation vorbereitet. Die bereitgestellte Medikamentendosis befindet sich in einem aufgelockerten Zustand. Es versteht sich, dass sich die Schutzkappe **950** nur herunterschwenken lässt, wenn sie zuvor bis zum Anschlag vorgezogen wurde. Die Bemessung des Mundstücks **900**, die Länge der Bügel **960** sowie die alleinige Möglichkeit, die Schutzkappe **950** nach unten zu schwenken, veranlasst den Patienten zwangsweise, den Inhalator lagegerecht anzusetzen. Bei einer irrtümlich kopfstehenden Verwendung des Inhalators, würde der Patient die Verdrehung sofort erkennen, da er mit seiner Nase an die Schutzkappe **950** stösst und so kaum das Mundstück **900** ansetzen kann.

Die Situation A3 kennzeichnet den Zustand, wenn sich die Schutzkappe **950** in der Schwenkbewegung befindet und noch nicht ihre unterste Lage erreicht hat.

### Figuren 2A bis 2D

Die Schutzkappe **950** besteht aus den beiden vorerwähnten Bügeln **960** sowie der eigentlichen Kappe **952**. An dem dem Mundstück **900** zugewandten Rand der Kappe **952** ist oben und unten mittig die Sichtfuge **968** vorgesehen, welche Raum für die Basisplatte **910** des Mundstücks **900** bieten.

Die beiden Bügel **960** erstrecken sich jeweils seitlich in die Kappe **952** hinein. An der vorderen Partie, welche in den Inhalator eingreift, besitzen die Bügel **960** einen speziellen, zueinander symmetrischen Aufbau. Jeder Bügel **960** weist einen quadratischen, abgerundeten Durchbruch **961**, einen darunterliegenden, nach innen gewandten Stift **962**, eine von der Bügelunterseite eingearbeitete Aussparung **963** mit einer Schnittkante **964** sowie vordere Abschrägungen **969** auf. Zwischen dem Durchbruch **961** und der Schnittkante **964** befindet sich in jedem Bügel **960** ein weiterer rechteckiger Durchbruch **970**. Versetzt über diesen Durchbruch **970** gibt es eine nach aussen weisende Mulde **971**. Eine ebensolche Mulde **972** ist im unteren Bereich des Bügels **960** nahe dem Eintritt in die Kappe **952** angeordnet.

### Figuren 3A bis 3D

Das Gehäuse-Unterteil **100** weist beiderseits mehrere, zueinander beabstandete und die Seitenwandung überragende Rastnocken **101** auf. Hinten, ist das Gehäuse-Unterteil **100** abschliessend verstärkt, so dass ein halbkreisförmiger Lagerring **102** entsteht. Beabstandet zum Lagerring **102** ist am Boden ebenfalls halbkreisförmig eine Doppelwandung **103** mit einer radialen Aufnahmenut **104** vorgesehen. Zwischen der Doppelwandung **103** verläuft mittig ein erhabener axialer Verbindungssteg **115**.

Am Boden sind zwei parallel verlaufende, sich von der Frontseite **105** erstreckende Balken **106** angeordnet, die im hinteren Bereich jeweils eine nach aussen weisende Einbuchtung **116** und im vorderen Bereich einen schräg geschnittenen Durchbruch **117** besitzen. Die Einbuchtung **116** begrenzt zusammen mit zwei sich im Abstand gegenüber stehenden Pfeilern **118** und einer an der Wandung des Gehäuse-Unterteils **100** verlaufenden Schiene **119** eine dellenartige Kugelpfanne **108**. In der Schiene **119** ist ebenfalls eine Einbuchtung **120** vorhanden, wobei sich die formgleichen Einbuchtungen **116,120** gegenüber liegen. Die Pfeiler **118** haben aufeinander gerichtete Spitzen **121**, die sich an der tiefsten Stelle in der Kugelpfanne **108** befinden. An den hinteren Pfeilern **118** ist jeweils ein nach innen weisender Haken **122** vorhanden. Vor den vorderen Pfeilern **118**, zur Frontseite **105** hin, ist jeweils ein erhabener Zapfen **123** angeordnet. An der Frontseite **105** sind zwei Aufnahmekerben **109** und im Boden-nahe jeder Gehäusewand - zwei axial verlaufende Längsschlitze **110** eingearbeitet. Am Eingang jedes Längsschlitzes **110** sitzt seitlich eine Sicherungsnocke **125**. Zwischen den beiden Balken **106** und der Frontseite **105** gibt es zwei U-förmige Vertiefungen **124.**

### Figuren 3E und 3F

In jeder Kugelpfanne **108** liegt im komplettierten Zustand eine Blockierkugel **130**, die sich in ordnungsgemässer Position des Inhalators an der tiefsten Stelle zwischen den Spitzen **121** der Pfeiler **118** befindet. Bei einer übermässigen horizontalen oder axialen Schrägstellung des Inhalators rollen die Blockierkugeln **130** in die Einbuchtung **116** des Balkens **106** bzw. in die Einbuchtung **120** der Schiene **119** und bewirken eine bei den Figuren 22A und 22B beschriebene Blockierung des Inhalators.

Zur Sicherstellung, dass das entnommene Mundstück **900** vor dem Schliessen des Inhalators replaziert wird, sind als Option auf die Zapfen **123** Blockierhaken **140** aufgesteckt. Ein Blockierhaken **140** besteht aus einem Federarm **142** und einem Hebel **143** mit einem seitlich abstehenden Blockierzacken **144** und der auf die Frontseite **105** zeigenden Schaltklinke **145**. Der Federarm **142** eines Blockierhakens **140** durchgreift den Durchbruch **117**, während von der Spannung des Federarms **142** der Hebel **143** nach aussen gedrückt wird, dass sein Blockierzacken **144** in den Verlauf des Längsschlitzes **110** hineinragt, wo im komplettierten Zustand der jeweilige Bügel **960** der Schutzkappe **900** sitzt. Die Funktion der Blockierhaken **140** ist bei den Figuren 21A bis 21C detailliert beschrieben.

### Figur 4

Das Gehäuse-Oberteil **150** weist komplementär zu den Rastnocken **101** an seinen Seitenwandungen Stecklöcher **153** auf. Analog zum Gehäuse-Unterteil **100** besitzt auch das Gehäuse-Oberteil **150** hinten abschliessend einen halbkreisförmigen Lagerring **154** sowie eine Doppelwandung **155** mit einer Aufnahmenut **156**. Die jeweils halben Lagerringe **102** und **154** und die Aufnahmenuten **104** und **156** ergänzen sich zu Vollkreisen.

An den Seitenwänden, der Aufnahmenut **156** vorgelagert, sind jeweils eine Stütznocke **158** und eine höhere Überspringrippe **157** vorgesehen. Die Stütznocke **158** und die Überspringrippe **157** ragen zur Mitte des Gehäuse-Oberteils **150** hin und entspringen der Seitenwand gemeinsam. Benachbart zum Fenster **152** sind zwei zueinander beabstandete, parallele Stützen **159** am Gehäuseboden angeordnet. Im Boden befindet sich ferner die Aussparung **151** für den einzusetzenden Trichter. Beiderseits dieser Aussparung **151**, zu den Seitenwänden hin, erhebt sich vom Gehäuseboden jeweils eine Begrenzungsnocke **164**.

Korrepondierend mit den Längsschlitzen **110** im Gehäuse-Unterteil **100** sind auch in der Frontseite **160** des Gehäuse-Oberteils **150** zwei Schlitze **161** vorhanden. In der Frontseite **160** befindet sich ausserdem eine zentrale Aufnahmekerbe **162** und von der Frontseite **160** - in Richtung des Mundstücks **900** - erstrecken sich zwei elastische Klemmzinken **163**. Zwischen der Frontseite **160** und dem Ansatz der Klemmzinken **163** sowie angrenzend an die Aufnahmekerbe **162** spannt sich ein Quersteg **165** auf. Hinter der Frontseite **160** gehen die Klemmzinken **163** in ein vertikales U-Profil **166** über, wobei die Vertikalnuten **167** der U-Profile **166** innerlich an die Frontseite **160** angrenzen und zueinander gewandt sind.

### Figuren 5A bis 5E

Das aus der Basisplatte **910** und dem Mundrohr **920** bestehende Mundstück **900** wird vorteilhafterweise aus zwei Hälften gebildet, die z.B. durch ein an der Stirnseite **921** vorgesehenes Filmscharnier **923** zusammenhängen. Auf der Basisplatte **910**, dem Inhalator zugewandt, befinden sich unten auf jeder Hälfte ein ganzer Steckzapfen **911** und oben ein Halbnocken **912**, der sich mit dem benachbarten Halbnocken **912** ergänzt. Jeder Steckzapfen **911** hat an seinem vorderen freien Ende, im unteren Bereich, eine Ausnehmung **930** mit einer nach innen weisenden Anschrägung **931**.

Unterhalb der beiden Halbnocken **912** ist eine Eingriffsöffnung **913** eingearbeitet, die sich als Schacht **932** bis in das Mundrohr **920** hinein erstreckt. Tiefer im Schacht **932** ist jeweils seitlich in der Wandung des Mundrohrs **920** eine vertiefte Rille **933** vorhanden. Unter der Eingriffsöffnung **913** liegt der Kanaleingang **914** für die Zerstäuberstrecke **924** des Mundrohrs **920**. Der Kanaleingang **914** ist über die Zerstäuberstrecke **924** mit dem Kanalausgang **922** verbunden. An der Basisplatte **910** unter dem Kanaleingang **914** ist jeweils eine sich mit der zweiten Mundstückhälfte ergänzende, horizontale Rampe **935** angeordnet.

Innerhalb der Zerstäuberstrecke **924** sind hinter dem Kanaleingang **914** mehrere in die Zerstäuberstrecke **924** hineinragende Schikanen **925** zum Aufprall und zur Verwirbelung des medikamenthaltigen Luftstromes vorgesehen, so dass die Zerstäuberstrecke **924** einen kurvenreichen Verlauf erhält. Näher dem Kanalausgang **922** tritt das durchfliessende Pulveraerosol in einer S-Kurve in einen vergrösserten Kanalabschnitt **928** ein und wird hier zum Kanalausgang **922** umgelenkt. Die besondere Gestaltung der Zerstäuberstrecke bezweckt die Pulverdesagglomeration und eine Reduktion der Pulverflussgeschwindigkeit zur Abscheidung von gröberen, inhalativ unwirksamen Partikeln. Zugleich wird somit das Impaktieren von Wirkstoffpartikeln im Rachenraum des Patienten verhindert. Zumindest im Bereich der Stirnseite **921** ist das Mundrohr **920** horizontal abgeflacht, so dass der Patient zur lagegerechten Positionierung des Inhalators beim Gebrauch veranlasst wird.

### Figuren 6A und 6B

Die Schieberschiene **200** besitzt zwei seitlich sich von der Stirnseite **201**, parallel zueinander erstreckende Längsnuten **202**, die etwa bis zur Hälfte der Schieberschiene **200** reichen. In Verlängerung der Stirnseite **201** erstrecken sich vom Boden der Schieberschiene **200** nach unten zwei zueinander beabstandete Füsse **204**. Oben besitzt die Schieberschiene **200** eine leistenförmige Dachpartie **210**, welche die Stirnseite **201** vordachartig überragt. Der Stirnseite **201** gegenüberliegend hat die Schieberschiene **200** als Abschluss eine Anschrägung **205**, die rampenförmig auf die Deckfläche der Schieberschiene **200** übergeht, wobei die Dachpartie **210** bereits vor der Anschrägung **205** endet.

### Figuren 7A bis 7D

Der Schlitten **500** besitzt zwei auf der Frontseite **501** beginnende, sich seitlich erhebende Flügel **503,523**, woran je ein nach aussen weisender Nocken **504** vorgesehen ist. In der Horizontalen ist der Nocken **504** breiter als in der Vertikalen, so dass sich etwa eine ovale Form ergibt. In die Flügel **503**,**523** ist ein im Bogen verlaufender Durchbruch **505** eingearbeitet, der den Nocken **504** von unten im Teilkreis umläuft. An seiner Hinterseite **507** weist der Schlitten **500** zwei sich seitlich erhebende Streben **508**,**510** auf, wobei die Strebe **508** eine horizontale Abkröpfung **509** hat. Von der Schlittenbasis **511** erheben sich zwei Ziehnocken **512**, während sich zur Hinterseite **507** die Schlittenbasis **511** in Form von zwei elastischen Federzungen **513** fortsetzt, die als Federkeile **514** enden und seitlich auslenkbar sind. Zwischen den beiden Federzungen **513** erstreckt sich mittig durch die Schlittenbasis **511** eine längs laufende Nut **520**. An der Frontseite **501** ist zwischen der Nut **520** und den Flügeln **503**,**523** jeweils eine Zugklinke **521** vorhanden. Zur Frontseite **501** hin besitzt der Flügel **523** innen einen Rasterabschnitt **515.**

Unterhalb der Nut **520** besitzt der Schlitten **500** an seiner Unterseite zwei Kufen **522**. Ebenfalls an der Schlittenunterseite, von den Kufen **522** gesehen nach aussen, sind korrespondierend zu den beiden Kugelpfannen **108** im Gehäuse-Unterteil **100** zwei Paare von Prallstegen **524** vorhanden. Die beiden Prallstege **524** eines Paares stehen zueinander im Abstand. Jedem Paar von Prallstegen **524** ist in Richtung der Frontseite **501** eine abgesetzte Auswölbung **516** vorgelagert.

### Figuren 8A bis 8C

Der zungenartige Dosierschieber **300** besitzt nahe seiner Vorderkante **301** als Durchgangsbohrung die Dosierkavität **302**. Von der Vorderkante **301** aus erstreckt sich der Dosierschieber **300** zunächst als schmale Zungenspitze **303** und erweitert sich dann zur Hinterpartie **304**. An der Aussenflanke der Hinterpartie **304** ist ein Federblatt **305** angeordnet, von dem sich eine Nocke **306** erhebt.

An der Unterseite weist der Dosierschieber **300** Flankenstege **307** auf, die unmittelbar hinter der Dosierkavität **302** ansetzen und dort Anschlagkanten **308** bilden. Die Dosierkavität **302** ist an der Unterseite von einem radialen Dichtkranz **320** umgeben. Nahe dem Übergang zur Hinterpartie **304** erstrecken sich zwei abwärtsweisende Quernocken **309**. An der Hinterkante **310** des Dosierschiebers **300** befinden sich zwei nach unten gerichtete, profilierte Sperrhaken **321**.

### Figur 9A und 9B

Der Zuhalter **400** hat die Funktion, das in der Dosierkavität **302** bereitgestellte Medikament, erst bei einer entsprechend kraftvollen Inhalation freizugeben. Zuvorderst besitzt der Zuhalter **400** eine hülsenförmige Verschlusspartie **401** mit der Durchlassöffnung **402**. Hinter der Verschlusspartie **401** setzen äussere, vertikale Seitenstege **403**, beginnend mit einem Anschlag **404**, an. An jedem Seitensteg **403** ist ein nach aussen weisender, rampenförmiger Flügel **405** angeordnet. In Richtung der Hinterpartie **406** folgt dem Flügel **405** ein Ausleger **407**. Unten an der Hinterkante **408** besitzt der Zuhalter **400** noch einen nach aussen gerichteten Mitnehmer **409.** Am Boden, im Bereich der Flügel **405,** verlaufen zwei aufeinandergerichtete Basisplatten **410,** die einen Durchgangsspalt **411** lassen. Das Ende der Hinterpartie **406** ist zur Hinterkante **408** hin mit einer Abdeckung **420** versehen, die sich oben über die beiden parallelen Seitenstege **403** spannt und etwa im Bereich der Ausleger **407** beginnt. In der Abdeckung **420** gibt es eine sich mittig und längs erstreckende, ausgerundete Nut **421**, die zum Zentrum des Zuhalters **400** offen ist.

### Figuren 10A und 10B

Das Ventilschild **800** hat die Funktion, den Patienten zu veranlassen, für eine ordentliche Inhalation einen bestimmten Mindestsog zu generieren. Das Ventilschild **800** besteht aus einer zylindrischen Kapsel **810** und mehreren daran ansetzenden Armen mit verschiedenen Aufgaben. Die Kapsel **810** weist einen die Öffnung umgebenden, nach aussen überstehenden, flanschartigen Kragen **811** auf. Der Boden **812** der Kapsel **810** ist nach aussen konvex gekrümmt und besitzt äusserlich zahlreiche erhabene Noppen **813**.

Senkrecht auf diesen Kragen **811** und in axialer Richtung sind die weiteren Elemente des Ventilschilds **800** aufgesetzt. Auf dem Kragen **811** sitzt zunächst ein Paar von gegenüberliegenden, langen Tentakeln **820,** die zuvorderst Mitnehmer **821** besitzen. Vor den Tentakeln **820** sind zwei kürzere Federarme **822** mit nach aussen gewandten Keilprofilen **823** an den Spitzen angeordnet. Hinter den Tentakeln **820** gibt es zwei weitere Federarme **825** mit nach aussen gewandten Haken **826**. Zwischen diesen Federarmen **825** befinden sich zwei nahe zusammenstehende, kurze Sperrzacken **824.**

### Figuren 11A und 11B

Die kapselförmige Ventilführung **850** dient - in das Gehäuse-Unterteil **100** und das Gehäuse-Oberteil **150** eingesetzt - dazu, das Ventilschild **800**, d.h. deren Kapsel **810**, aufzunehmen. Die Ventilführung **850** hat insoweit die Funktion eines Gleitlagers. Komplementär zum Kragen **811** des Ventilschilds **800** besitzt die Ventilführung **850** einen äusseren Anschlagflansch **851**. Gleitrippen **852** im Innern der Ventilführung **850** bezwecken eine Verminderung der Reibung beim Ausfahren des Ventilschilds **800**. Der perforierte Boden **854** weist zahlreiche Löcher **855** auf, so dass die Noppen **813** des Ventilschilds **800** darin Platz finden. Oben und unten gibt es im Anschlagflansch **851** zwei sich diametral gegenüber stehende Kerben **856**. Die Löcher **855** und die Noppen **813** ermöglichen es, den Inhalator rückseitig quasi geschlossen zu gestalten und somit das Eindringen von Schmutzpartikeln sowie das unbeabsichtigte Verschieben des Ventilschilds **800** zu vermeiden.

### Figuren 12A und 12B

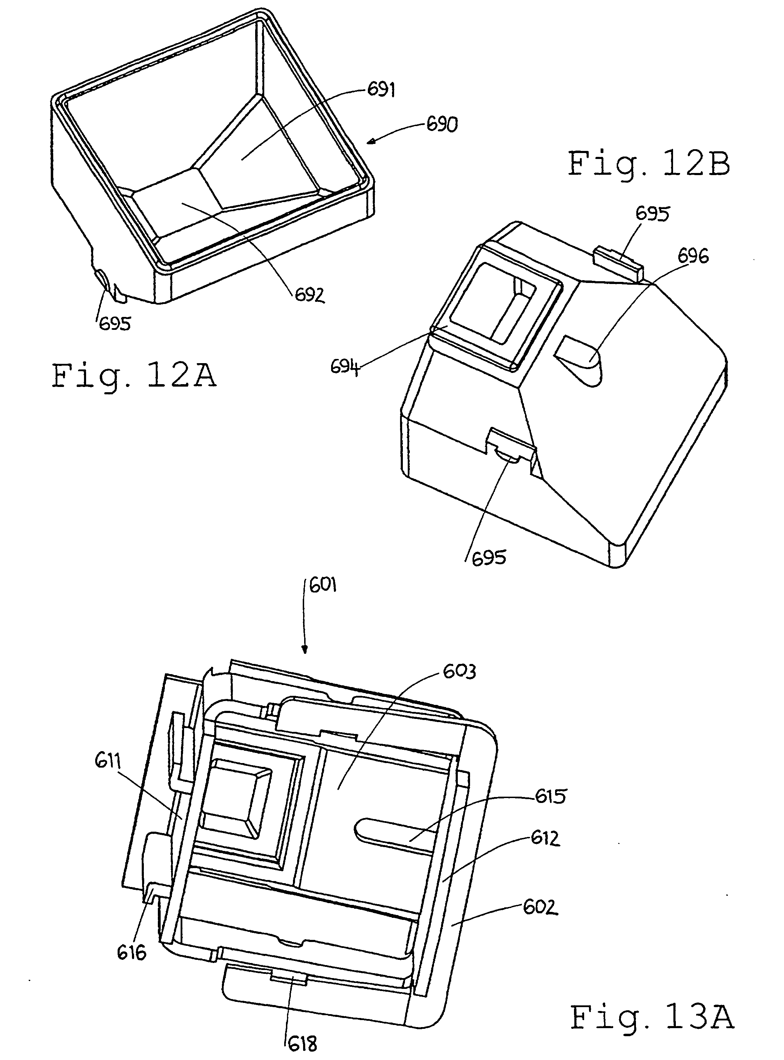

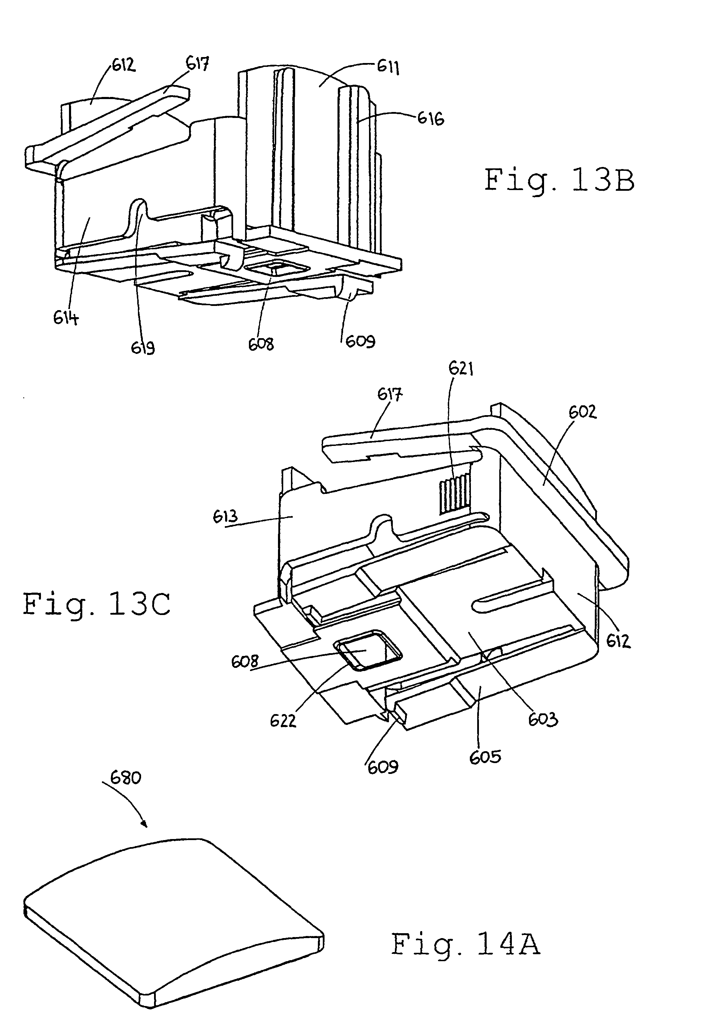

Der Trichter **690** ist zum Einsetzen in die Trichterfassung **601** vorgesehen (s. Figuren 13A, 13B). Der Trichterboden **691** ist zum Auslass **692** hin schräg gestaltet, damit das Medikamentenpulver günstig abfliesst. Der Auslass **692** ist äusserlich von einem Dichtungselement **694** umgeben. Äusserlich weist der Trichter **690** auf zwei gegenüber liegenden Seiten überstehende Haltenocken **695** sowie eine zentral positionierte Fixiernase **696** auf, die auf dem schrägen Trichterboden zwischen den Haltenocken **695** sitzt.

### Figuren 13A und 13B

Die kastenförmige Trichterfassung **601** besitzt unten den Fassungsboden **603**, die Vorderwand **611**, die Rückwand **612** sowie die zwei zwischen der Vorderund der Rückwand **611**,**612** liegenden, halbhohen Seitenwände **613**,**614**. Im Fassungsboden **603** befindet sich der Trichterauslass **608** sowie eine längliche Nut **615**. Zuunterst umgibt eine Dichtung **622** den Trichterauslass **608**, damit der Eintritt von Feuchtigkeit und der Austritt von Pulver aus dem Trichter **690** auf die Gleitflächen des Dosierschiebers **300** verhindert werden.

An der Vorderwand **611** sind zwei Winkelschienen **616** senkrecht angeordnet und an der Rückwand **612** befindet sich oben ein Stützrand **602**, von dem sich je ein Federarm **617** entlang der beiden Seitenwände **613**,**614** erstreckt. In den Federarmen **617** ist je eine auf die jeweilige Seitenwand **613**,**614** weisende Nut **618** vorhanden. Die Seitenwände **613**,**614** besitzen etwa in ihrer Mitte an der Unterkante jeweils eine nach unten offene Kerbe **619**. An der einen Seitenwand **613** ist nahe der Rückwand **612** ein Rasterabschnitt **621** vorgesehen. An den Aussenflanken des Fassungsbodens **603** erstrecken sich zwei in der Rückwand **612** mündende, elastische Lamellen **605**, an deren vertikal beweglichen Enden nach unten ragende Blockiernocken **609** vorgesehen sind.

### Figuren 14A bis 14C

Der Trichterdeckel **680** dient zum Verschliessen des Trichters **690**. An der Unterseite des Trichterdeckels **680** befindet sich eine Kammer **681**, die mit einer semipermeablen Membran **682** verschlossen ist. Die Kammer **681** ist zur Aufnahme eines Feuchte anziehenden Trocknungspulvers vorgesehen, welche durch die semipermeable Membran **682** diffundieren kann.

### Figuren 15A und 15B

Im Zustand der komplettierten Trichteranordnung ist der Trichter **690** in die Trichterfassung **601** eingesetzt und der Trichter **690** mit dem Trichterdeckel **680** verschlossen. Der Ablauf der Montage des Inhalators muss aber nicht die vorherige Komplettierung der Trichteranordnung beinhalten.

### Figuren 16A und 16B

Der komplette Zähler **700** besteht aus dem Einerrad **701,** dem Zehnerrad **780,** dem Hunderterrad **720**, dem Zählerstock **740**, der Zählerdeckplatte **760** sowie zwei hier nicht sichtbaren gleichen Triebrädern. Zweck des Zählers **700** ist es, die Anzahl der verbrauchten bzw. noch verfügbaren Dosen zu registrieren und dem Patienten die momentan erfolgte Inhalation anzuzeigen, sofern diese ordnungsgemäss erfolgte. Am Umfang der auf die Achse **741** des Zählerstocks **740** aufgesteckten Zählerräder **701**, **780** und **720** sind Zahlen und eventuell eine Farbmarkierung aufgetragen. Der geltende Zählerstand wird unter einer Linse **742** angezeigt, die im Fenster **152** des Gehäuse-Oberteils **150** sitzt. Die Linse **742** ist mit dem Zählerstock **740** verbunden.

### Figuren 16C und 16D

Das Einerrad **701** besitzt an seiner Aussenfläche **702** zehn radial verteilte Nocken **703.**

### Figuren 16E und 16F

Das Zehnerrad **780** ist mit seinem inneren Zahnkranz **781** an sich von konventioneller Bauart.

### Figuren 16G und 16H

Das Hunderterrad **720** weist ebenfalls einen inneren Zahnkranz **721** sowie eine nach aussen ragende Schlussnocke **722** auf.

### Figuren 161 und 16J

Der Zählerstock **740** besteht aus der Basisplatte **743,** der oben rechtwinklig angesetzten Linse **742**, der sich von der Basisplatte **743** senkrecht erstreckenden Achse **741** sowie dem Triebradlager **744**.

### Figuren 16K und 16L

Anliegend auf dem Einerrad **701** ist auf der Achse **741** des Zählerstocks **740** die Zählerdeckplatte **760** fixiert. Die Zählerdeckplatte **760** besitzt eine elastische Stellzunge **761** mit einer Keilnocke **762** am Ende, welche sich stets zwischen zwei Nocken **703** des Einerrads **701** schiebt.

### Figuren 16M und 16N

Das sternförmige Triebrad **790** wird einmal zwischen dem Einerrad **701** und dem Zehnerrad **780** sowie einmal zwischen dem Zehnerrad **780** und dem Hunderterrad **720** eingesetzt. Es weist sechs gleichmässig angeordnete Zähne **791** auf, wovon an ihrer Spitze auf einer Seite des Triebrads **790** jeder zweite Zahn **791** eine Hinterschneidung **792** besitzt.

### Figur 160

Wenn eine ordentliche Inhalation vollendet wurde und der Inhalator durch Hochschwenken und Einschieben der Schutzkappe **950** wieder geschlossen wird, erfolgt die Betätigung des Zählers **700.** Nur beim Zurückschieben des Dosierschiebers **300** in die Ausgangslage - *Situation A1* - wird von der auf dem Federblatt **305** befindlichen Nocke **306** eine Nocke **703** auf dem Einerrad **701** erfasst und dadurch das Einerrad **701** um eine Zählstellung weitergedreht.

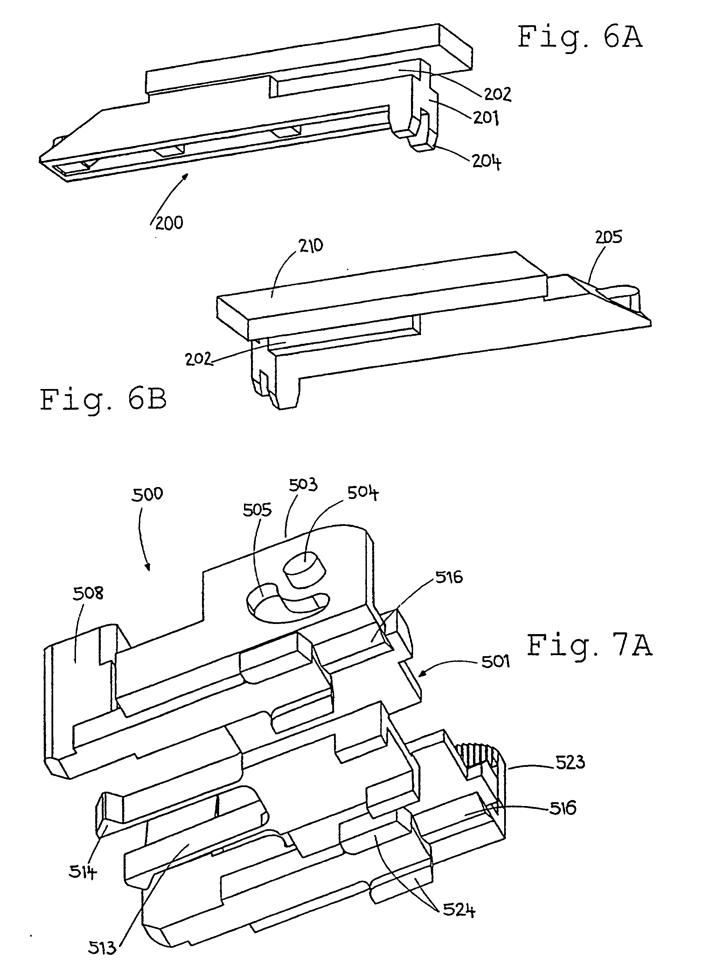

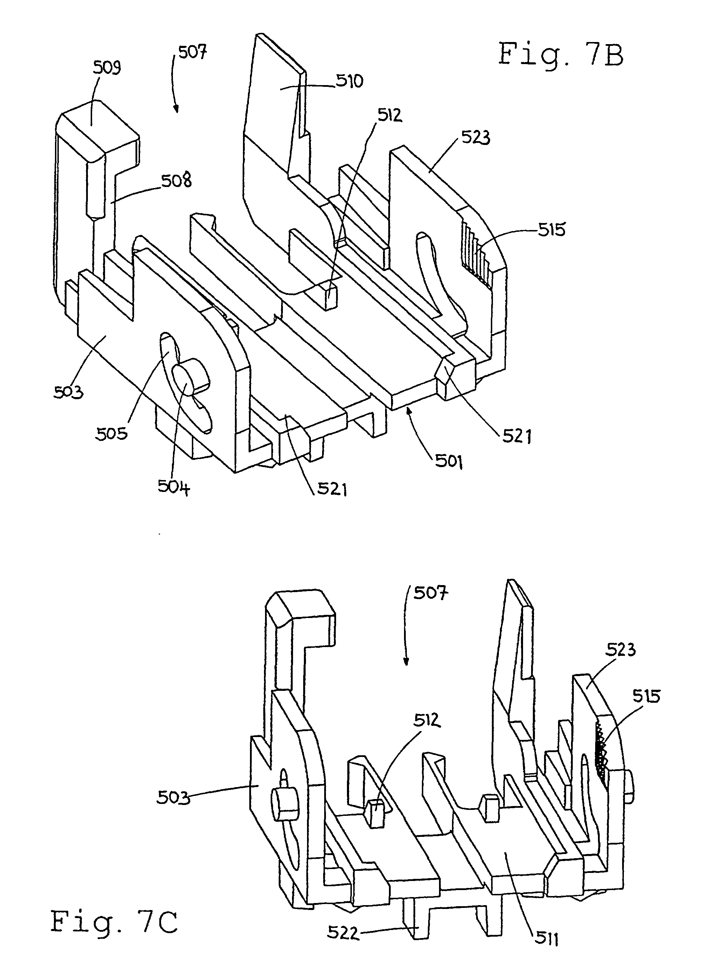

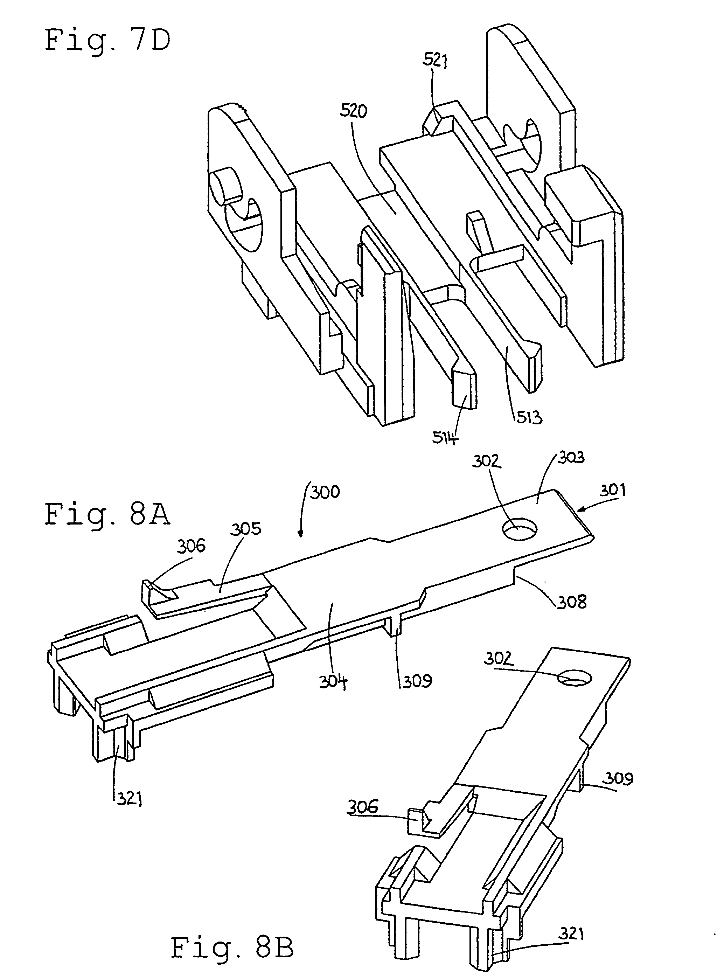

Wenn dem Inhalator die vorgesehene Dosenzahl entnommen wurde, ist das Hunderterrad **720** in einer solchen Stellung, dass sich die Schlussnocke **722** weit oben positioniert hat und beim Vorziehen des Schlittens **500** die Abkröpfung **509** (s. Figuren 7A bis 7D) an die Schlussnocke **722** anschlägt. Damit ist die weitere Betätigung des Inhalators blockiert.

### Figuren 17A bis 17C

Als Option zur Erhöhung der Sicherheit bei der Handhabung des Inhalators sind zwei in das Gehäuse-Unterteil **100**, auf die Zapfen **123** aufsteckbare, drehbewegliche Blockierhaken **140** vorgesehen. Der Blockierhaken **140** ist zweiarmig und gliedert sich in einen dünnen Federarm **142** sowie einen kompakteren Hebel **143** auf, wobei sich der Federarm **142** vom Hebel **143** abspreizt. Im Blockierhaken **140** ist eine Bohrung **146** vorhanden, so dass man den Blockierhaken **140** auf den Zapfen **123** aufstecken kann. Am Hebel **143** ist ein zur Seite hinausragender und vom Federarm **142** wegweisender Blockierzacken **144** sowie eine nach vorn zeigende, den Hebel **143** verlängernde Schaltklinke **145** vorhanden.

### Figuren 18A bis 18C

Im zusammengebauten Zustand ergibt sich in der *Situation A1* folgende Anordnung. Das Gehäuse-Unterteil **100** und das Gehäuse-Oberteil **150** sind zusammengefügt. Von vorn ist das Mundstück **900** eingesteckt und die Schutzkappe **950** völlig geschlossen.

In den Gehäuseteilen **100**, **150** liegen die Schieberschiene **200**, der Schlitten **500**, der Dosierschieber **300**, der Zuhalter **400**. Eingesetzt sind die Ventilführung **850** und darin das Ventilschild **800** sowie die komplette Trichtervorrichtung - bestehend aus Trichter **690**, Trichterfassung **601** und Trichterdeckel **680** - und der Zähler **700**. Das Ventilschild **800** ist in seiner hintersten Position, und der Dosierschieber **300** steht so, dass sich die Dosierkavität **302** unter dem Trichterauslass **608** positioniert, mit Medikament füllen kann. Die Verschlusspartie **401** des Zuhalters **400** ragt in den Kanaleingang **914** hinein. Die Klemmzinken **163** sitzen - das Mundstück **900** zusätzlich fixierend - in dessen Schacht **932** und greifen in die Rillen **933** ein. Die zusammengefügten Halbnocken **912** des Mundstücks **900** sind in die Aufnahmekerbe **162** im Gehäuse-Oberteil **150** eingerastet. Die Steckzapfen **911** des Mundstücks **900** durchdringen die Aufnahmekerben **109** im Gehäuse-Unterteil **100**, und die Rampe **935** des Mundstücks **900** untergreift die Vorderkante der Dachpartie der Schieberschiene **200**. Die Bügel **960** der Schutzkappe **950** ragen durch die Schlitze **110**,**161** im Gehäuse-Unterteil **100** bzw. im Gehäuse-Oberteil **150** und umfassen die Flügel **503,523** des Schlittens **500**. Hierbei hängen die Stifte **962** in den Durchbrüchen **505**, während die Nocken **504** in die Durchbrüche **961** eingreifen. Zur Fixierung der Schutzkappe **950** in der Ausgangsposition sind die Sicherungsnocken **125** in die Mulden **972** eingerastet.

Die Füsse **204** der Schieberschiene **200** stecken in den Vertiefungen **124** im Gehäuse-Unterteil **100**. Die Winkelschienen **616** der Trichterfassung **601** sind in die Vertikalnuten **167** der U-Profile **166** am Gehäuse-Oberteil **150** eingefahren. Der Trichter **690** sitzt mit seinen Haltenocken **695** und seiner Fixiernase **696** in den Kerben **619** bzw. in der Nut **615** der Trichterfassung **601**. Der Auslass **692** des Trichters **690** mit dem Dichtungselement **694** befindet sich im Trichterauslass **608**. Zusätzlich wird die Trichterfassung **601** seitlich von den Begrenzungsnocken **164** im Gehäuse-Oberteil **150** fixiert.

Von den Stützen **159** im Gehäuse-Oberteil **150** wird der komplette Zähler **700** gehalten. Die Kapsel **810** des Ventilschilds **800** sitzt maximal in der Ventilführung **850**, wobei deren Anschlagflansch **851** in den Aufnahmenuten **104,156** des Gehäuse-Unterteil **100** bzw. des Gehäuse-Oberteil **150** sitzt und der Verbindungssteg **115** mit der Kerbe **856** in Eingriff kommt.

### Figuren 19A bis 19D

Diese Figurenfolge veranschaulicht die Freigabe des Zuhalters **400**, der die mit Medikament gefüllte Dosierkavität **302** des Dosierschiebers **300** umschliesst, beim Abschwenken der Schutzkappe **950.**

### Figuren 19A und 19B

Gemäss *Situation A1* ist der Schlitten **500** so positioniert, dass seine Flügel **503,523** vor der Trichterfassung **601** stehen, d.h. der in den Durchbruch **505** eingreifende Stift **962** vom Bügel **960** der Schutzkappe **950** ist hinsichtlich der Entriegelung des unbeweglichen Zuhalters **400** wirkungslos. Die Blockiernocken **609** unter der Trichterfassung **601** hinterfassen die am Zuhalter **400** seitlich abstehenden Flügel **405**. Die Dosierkavität **302** befindet sich unterhalb des Trichterauslasses **608** und könnte bereits mit Medikament gefüllt sein.

### Figur 19B

Die Schutzkappe **950** wurde inzwischen vollständig abgezogen und damit der an den Bügeln **960** hängende Schlitten **500** vorgezogen; es war die *Situation A2* erreicht. Der Zuhalter **400** ist noch unbeweglich und umschliesst mit seiner Verschlusspartie **401** die durch das Abziehen der Schutzkappe **950** in den Kanaleingang **914** des Mundstücks **900** vorgeschobene und mit Medikament gefüllte Dosierkavität **302**.

Nun setzte das Abwärtsschwenken der Schutzkappe **950** ein, d.h. die *Situation* **A3** wird durchfahren. Jedoch ist die Schutzkappe **950** noch nicht gänzlich heruntergeschwenkt, so dass mit der Abschwenkbewegung der Stift **962** im Durchbruch **505** aufsteigt und folglich sukzessive die den Zuhalter **400** arretierende Lamelle **605** anhebt und entriegelt.

### Figur 19D

Gemäss der erreichten *Situation A3* - dies gilt auch für die *Situationen A4 bis* A7 - ist die Schutzkappe **950** völlig heruntergeschwenkt, wodurch der Stift **962** die Lamelle **605** hochdrückt. Somit ist der Blockiernocken **609** mit dem Flügel **405** am Zuhalter **400** ausser Eingriff. Der Zuhalter **400** ist beweglich, d.h. im Anschluss an die *Situation A3* besteht Bereitschaft zur Inhalation. Die heruntergeschwenkte Schutzkappe **950** wird in dieser Stellung durch das Zusammenwirken der Sicherungsnocken **125** im Gehäuse-Unterteil **100** und den Mulden **971** in den Bügeln **960** fixiert.

### Figuren 20A bis 20F

Diese Figurenfolge veranschaulicht einen gänzlich ablaufenden Inhalationszyklus mit den mechanischen Abläufen in den verschiedenen möglichen Situationen.

### Figur 20A

Gemäss der *Situation A1* befinden sich das Ventilschild **800,** der Schlitten **500** und der Zuhalter **400** in ihrer hinteren Endlage. Dies ist der Zustand des Inhalators nach dem Schliessen der Schutzkappe **950** im Anschluss an eine ordnungsgemäss durchgeführte Inhalation bzw. vor dem ersten Gebrauch. Mit dem Einschieben der Schutzkappe **950** wurde vom Schlitten **500** der Zuhalter **400**, das Ventilschild **800** und der Dosierschieber **300** in die hintere Endlage zurückgeschoben. Der Schlitten **500** erfasst mit seiner Zugklinke **521** den Mitnehmer **409** des Zuhalters **400**. Mit seinen Federkeilen **514** drückt der Schlitten **500** gegen die Sperrhaken **321** des Dosierschiebers **300**, wobei die Federkeile **514** nach innen von den Sperrzacken **824** umschlossen sind.

Die beiden Streben **508,510** des Schlittens **500** haben das Ventilschild **800** in seine Ausgangslage geschoben. Von der Nocke **306** am Dosierschieber **300** wurde eine Nocke **703** am Einerrad **701** des Zähler **700** um eine Einheit weiter gestellt. Die Dosierkavität **302** befindet sich nun wieder unter dem Trichterauslass **608.**

### Figur 20B

Gemäss *Situation A4* besteht hier die Bereitschaft zur Inhalation. Durch das Herausziehen der Schutzkappe **950** ist das Ventilschild **800** aus der hintersten Position vorgerückt. Die Mitnehmer **821** an den Tentakeln **820** wurden von den Flügeln **503,523** erfasst und geringfügig vorgezogen, so dass die Noppen **813** des Ventilschilds **800** aus den Löchern **855** der Ventilführung **850** entfernt sind und Luftspalte entstehen. Durch diese Luftspalte kann der inhalierende Patient seine Atemluft ziehen, falls nicht noch andere Lufteinlässe am Inhalator vorgesehen werden. Beim Herausziehen der Schutzkappe **950** wurde der Schlitten **500** mit seinem Rasterabschnitt **515** am Rasterabschnitt **621** der Trichterfassung vorbeibewegt, so dass eine Vibration zur Begünstigung des Fliessens des Medikamentenpulvers aus dem Trichter **690** in die Dosierkavität **302** erzeugt wurde. Die Rasterabschnitte **515,621** sind so bemessen und angeordnet, dass beim Abziehen der Schutzkappe **950** nur solange Vibrationen erzeugt werden, wie sich die Dosierkavität **302** unter dem Trichterauslass **608** befindet. Beginnt der Schlitten **500** den Dosierschieber **300** mitzuziehen, kommen die Rasterabschnitte **107** und **515** ausser Eingriff.

Ferner wurde der Dosierschieber **300** von den Ziehnocken **512** des Schlittens **500** an den Quernocken **309** erfasst und in Richtung Mundstück **900** so weit nach vorn bewegt, dass die Dosierkavität **302** nun von der Verschlusspartie **401** des Zuhalters **400** umschlossen ist. Der Zuhalter **400** ist auch freigegeben, da die Blockiernocken **609** unterhalb der Trichterfassung **601** von den Flügeln **405** des Zuhalters **400** mit dem Herunterschwenken der Schutzkappe **950** abgehoben haben. Die Keilprofile **823** der Federarme **822** des Ventilschilds **800** stehen an den Überspringrippen **157** des Gehäuse-Oberteils **150** an.

Auf die Federarme **617** der Trichterfassung **601** wird von oben Druck ausgeübt, so dass alle darunter liegenden Bauteile einer gewissen Flächenpressung ausgesetzt sind. Damit erhöht man die Dichtheit und verhindert, das Austreten von Medikamentenpulver. Nach dem Herunterschwenken der Schutzkappe **950** besteht die Bereitschaft zur Inhalation und jetzt ist die leichtere Beweglichkeit des Zuhalters **400** erwünscht. Beim Abschwenken der Bügel **960** wird die von oben wirkende Flächenpressung zum Teil kompensiert, denn der im Durchbruch **505** aufstrebende Stift **962** drückt gegen die Lamellen **605**. Durch die ovale Form des Nockens **504** und die Geometrie des Durchbruchs **961** hat der Nocken **504** ein gewollt grösseres vertikales Spiel im Durchbruch **961** als sein horizontales Spiel. Die verminderte Flächenpressung ergibt nun eine leichtere Beweglichkeit des Zuhalters **400** bei der Inhalation.

### Figur 20C

Gemäss *Situation A5 -* der Inhalator wird nach unterlassener Inhalation wieder geschlossen - blieb das Ventilschild **800** in seiner Position, d.h. es wurde nicht nach vorn gesaugt. Beim Aufstecken der Schutzkappe **950** wird der Schlitten **500** zurückgeschoben; seine Federzungen **513** weichen den Sperrhaken **321** des Dosierschiebers **300** aus. Vom Schlitten **500** wird das Ventilschild **800** wieder in seine hinterste Position gedrückt; der Zuhalter **400** wird wieder verriegelt. Der Dosierschieber **300** bleibt mit seiner gefüllten Dosierkavität **302** aber in seiner vorderen Position; er verharrt infolge adäquater Reibung.

### Figuren 20D

Gemäss *Situation A6 -* die Inhalation wurde unvollendet abgebrochen - hat das Ventilschild **800** noch nicht seine vordere Position erreicht, wodurch der Zuhalter **400** noch nicht verschoben wurde, und die Medikamentendosis blieb umschlossen. Beim Aufstecken der Schutzkappe **950** und Zurückschieben des Schlittens **500**, bleibt der Dosierschieber **300** mit seiner ungeleerten Dosierkavität **302** vorn stehen. Die Federzungen **513** des Schlittens **500** stossen mit den Federkeilen **514** gegen die Sperrhaken **321** und werden somit nach innen gebogen. Hierdurch stossen die Federkeile **514** auf die Sperrzacken **824** und schieben somit das Ventilschild **800** zurück, bis das weitere Zurückschieben des Ventilschilds **800** von den beiden Streben **508** und **510** des Schlittens **500** erfolgt.

### Figur 20E

Nach unvollendeter Inhalation und wieder geschlossenem Inhalator - *Situation A8* - steht der Dosierschieber **300** gefüllt vorn, während Ventilschild **800** und Schlitten **500** sich wieder in der hinteren Ausgangslage befinden.

### Figur 20F

Gemäss *Situation A7* - nach vollendeter Inhalation - ist die Schutzkappe **950** vollständig heruntergeschwenkt, wodurch die Stifte **962** die Lamellen **605** der Trichterfassung **601** angehoben haben und die Flügel **405** des Zuhalters **400** entriegelt wurden. Während einer ordentlichen Inhalation ist das Ventilschild **800** nach vorn gesogen worden. Vom vorfahrenden Ventilschild **800** haben die Federarme **822** mit den Keilprofilen **823** die Überspringrippen **157** im Gehäuse-Oberteil **150** überwunden. Vom nach vorn laufenden Ventilschild **800** wurde der Zuhalter **400** bis in seine vordere Endlage geschoben, wodurch die Dosierkavität **302** frei und das Medikament vom Patienten inhaliert wurde.

Während einer ordentlichen Inhalation ist das Ventilschild **800** nach vorn gesogen worden, nachdem seine Federarme **822** mit dem Keilprofil **823** die Überspringrippen **157** überwunden haben. Mit der Geometrie des Keilprofils **823** und der Überspringrippen **157** sowie der Elastizität der Federarme **822** kann man die notwendige Sogleistung definieren. In der vordersten Position des Ventilschilds **800** sind die beiden elastischen Federarme **825** mit den daran befindenden Haken **826** hinter die im Gehäuse-Unterteils **100** angeordneten Haken **122** gefahren. Damit wird das selbsttätige Zurückgleiten des Ventilschilds **800** verhindert.

Bei der Rückwärtsbewegung des Schlittens **500** sitzen seine Federkeile **514** eingeklemmt zwischen den Sperrhaken **321** und den Sperrzacken **824** und können somit nicht entweichen. Damit werden jetzt der Dosierschieber **300** und das Ventilschild **800** von den Federkeilen **514** und den Streben **508,510** in die Ausgangsposition - *Situation A1* - zurückgeschoben. Hierbei lösen sich die Haken **826,122** voneinander.

### Figur 21A bis 21C

Diese Figurenfolge veranschaulicht die Funktion der eingesetzten Blockierhaken **140** im Zusammenspiel mit dem Mundstück **900** und den Bügeln **960** der Schutzkappe **950.**

### Figur 21A

In der *Ausgangsposition B1* ist die Schutzkappe **950** geschlossen, d.h. aufgeschoben; das Mundstück **900** fehlt jedoch beim Einsetzen der Blockierhaken **140**. Die Federarme **142** durchragen die Durchbrüche **117** in den Balken **106**, stützen sich darin ab und drücken die Hebel **143** nach aussen. Die Blockierzacken **144** stossen auf die undurchlässigen Bügel **960** der Schutzkappe **950.**

### Figur 21B

Hier - in der *Fehlposition B2* - ist die Schutzkappe **950** abgezogen und nach unten abgeschwenkt; der Schlitten **500** ist nach vorn gezogen, so dass vom Schlitten **500** die Federarme **142** in die Durchbrüche **117** zurück gedrückt werden, wodurch die Hebel **143** unter erhöhte Spannung geraten. Das Mundstück **900** ist aber z.B. für einen Reinigungsvorgang entfernt worden. Jetzt greifen die Blockierzacken **144** in die in den Bügeln **960** vorhandenen Durchbrüche **970** ein, denn vom Druck der Federarme **142** werden die Hebel **143** nach aussen gedrückt. In diesem Zustand kann die Schutzkappe **950** nicht hochgeschwenkt werden, um sie einzuschieben. So wird das Fehlen des Mundstücks **900** offensichtlich, und es ist ausgeschlossen, dass der Patient den Inhalator ohne eingesetztes Mundstück **900** einpackt und dann im Ernstfall nicht gebrauchen kann.

### Figur 21C

In der *Sollposition B3* ist das Mundstück **900** eingesetzt. Dabei ragen die Steckzapfen **911** des Mundstücks **900** in die Aufnahmekerben **109** hinein. Die Anschrägungen **931** der Steckzapfen **911** drücken hierbei, gegen die Spannung der Federarme **142**, die Schaltklinken **145** der Hebel **143** nach innen, so dass die Blockierzacken **144** aus den Durchbrüchen **970** herausgezogen sind. Somit kann die Schutzkappe **950** wieder hochgeschwenkt und geschlossen werden.

### Figuren 22A und 22B

Bei ordnungsgemässer Positionierung des Inhalators, d.h. eine überkritische Neigung in der Horizontalen oder in der axialen Drehachse liegt nicht vor, plazieren sich die Blockierkugeln **130** mittig der Kugelpfannen **108** in den tiefsten Stellen. In einer solchen Position kann man die Schutzkappe **950** ausziehen, da der angehängte Schlitten **500** nicht blockiert wird und auch ausfahrbar ist.

Ist die Neigung überkritisch, so rollen die Blockierkugeln **130** von den tiefsten Stellen an die seitlichen Begrenzungen **116,120** und liegen nun wegen der Schrägen in den Kugelpfannen **108** höher. Ein Herausfahren des Schlittens **500** wird jetzt blockiert. Die Blockierkugeln **130** geraten nun mit den Prallstegen **524** und den Auswölbungen **516** in Kollision, so dass letztlich die Schutzkappe **950** nicht abgezogen werden kann. Diese Sicherheitsvorkehrung gewährleistet ein lagegerechtes Halten des Inhalators beim Öffnen, so dass eine ordnungsgemässe Füllung der Dosierkavität **302** mit Medikamentenpulver gesichert ist. Der Inhalator muss in der vorgeschriebenen Position geöffnet werden, jedoch ist der Inhalator nach dem Öffnen in jeder Lage benutzbar, also insbesondere auch für liegende Patienten. Eine weitere Gebrauchskontrolle ergibt sich, indem die Schutzkappe **950** nur nach unten abschwenkbar ist.

### Figuren 23A bis 23G

Diese Figurenserie vermittelt einen Eindruck vom sukzessiven Aufbau des Inhalators, wobei keine Übereinstimmung mit der Montagefolge in der Massenfertigung bestehen muss.

In das Gehäuse-Unterteil **100** ist hinten die Ventilführung **850** und darin das Ventilschild **800** eingesetzt. Vorn steckt das Mundstück **900**, wobei zur besseren Veranschaulichung nur eine Hälfte des Mundstücks **900** dargestellt ist (Figur 23A). Der Inhalator ist mit der nahe dem Mundstück **900** positionierten Schieberschiene **200** und dem am Ventilschild **800** anschlagenden Schlitten **500** ergänzt (Figur 23B). Auf die Schieberschiene **200** ist nun der Dosierschieber **300** aufgesetzt (Figur 23C). In weiterer Komplettierung ist der Zuhalter **400** hinzugekommen (Figur 23D). Jetzt ist die Schutzkappe **950** mit den seitlichen Bügeln **960**, welche am Schlitten **500** angehängt sind, eingesetzt (Figur 23E). In zwei Ansichten (23F, 23G) werden hier die komplett eingesetzte Trichteranordnung **600**, welche der Schutzkappe **950** zugewandt ist, und der Zähler **700** gezeigt. Schliesslich müsste noch das Gehäuse-Oberteil **150** ergänzt werden.

Zum vorbeschriebenen Inhalator sind weitere, konstruktive Variationen realisierbar. Hier ausdrücklich erwähnt seien noch:
- Anstelle der Mulden **972** zur Arretierung der Schutzkappe **950** im eingeschobenen Zustand - *Situation A1* - könnte man nahe dem Eintritt in die Kappe **952** an der Oberseite der Bügel **960** je eine nach aussen weisende Nocke vorsehen, die im geschlossenen Zustand in Schlitze in der Frontseite **160** des Gehäuse-Oberteils **150** eingreifen. Um die Schutzkappe **950** vom Mundstück **900** abzuziehen, ist dann die Schutzkappe im Bereich der seitlichen Griffkonturen **951** zusammenzudrücken, wodurch sich die Nocken aushängen.
- Im Mundstück **900** kann man nahe dem Kanalausgang **922**, im vergrösserten Kanalabschnitt **928** einen räumlich, oberflächenprofilierten Wandabschnitt mit einer Querrillung vorsehen, um die Pulverdesagglomeration sowie die Abscheidung von gröberen, inhalativ unwirksamen Partikeln zu fördern.
- Zum Zusammenhalt der beiden Hälften des Mundstücks **900** könnte man an den inneren Schnittflächen der beiden Hälften jeweils zueinander komplementäre Verbindungselemente anordnen - z.B. eine Kombination aus Bohrungen und Nocken -, um beide Hälften nach visualisierbarer Reinigung und Trocknung wieder zusammenzustecken.
- Zur Einbettung der Trichteranordnung im Inhalator könnte man einen auf die Trichterfassung **601** aufgeschobenen Kragen aus elastischem Material einsetzen.
- Das im Trichter **690** bevorratete pharmakologische Trockenpulver kann einerseits lose Form besitzen. Einbezogen sind jedoch auch vordosierte Spendereinheiten, z.B. als extrudierter Pelletstrang oder in Perlenkettenform. Einzeldosierte Spendereinheiten könnten in Blistern oder auf Taperollen angeordnet sein. Es versteht sich, dass das Medikamentenreservoir und eine Vorrichtung, um die Einzeldosen abzuteilen, entsprechend zu gestalten ist.
- Abgesehen vom höheren herstellungstechnischen Aufwand, könnte das zuvor beschriebene zweiteilige Gehäuse, bestehend aus Gehäuse-Unterteil **100** und Gehäuseoberteil **150**, auch einstückig sein.
- Die Zerstäuberstrecke **924** im Mundstück **900** ist als gerader oder gewundener Kanal ausgebildet, in dem zumindest eine Schikane **925** angeordnet ist, wobei diese eine hineinragende Lamelle, eine Wand, ein Strömungskörper oder ein Sieb sein kann.
- Anstelle des mechanischen Zählers **700** ist auch ein Chip einsetzbar, mit welchem alle relevanten Daten, wie Anzahl der erfolgten Inhalationen, Zeitpunkt der Einnahme und Flussparameter erfasst werden.
- Die jetzt mittels der Überspringrippe **157** und der Federarme **822** realisierte Flusssteuerung könnte auch durch einen veränderbaren Widerstand innerhalb der Ventilführung **850** erfolgen.
- Zur Regulierung der Flussgeschwindigkeit ist es vorteilhaft, innerhalb des Mundrohrs **920**, nämlich am Beginn der Zerstäuberstrecke **924** oder dem Mundrohr vorgelagert, einen Einsatz zur Aufnahme einer wählbaren Düse vorzusehen.
- Zur gezielten Veränderung des Strömungswiderstands im Inhalator während einer Inhalation, der sich durch den Luftspalt zwischen der feststehenden Ventilführung **850** und der ausfahrenden Kapsel **810** des Ventilschilds **800** ergibt, kann man diesen bei der jeweiligen Position des Ventilschilds **800** wirksamen Luftspalt, inkremental durch körperliche Ungleichmässigkeiten an der Oberfläche der Kapsel **810** und/oder im Inneren der Ventilführung **850** gestalten. Hierfür kommen beispielsweise erweiternde bzw. verengende körperliche Abmessungen an der Kapsel **810** des Ventilschilds **800** bzw. in der Ventilführung **850** oder sich über deren Länge querschnittsveränderte Nuten in Betracht.
- Beim vorbeschriebenen Inhalator, aber auch bei Inhalatoren generell, besteht die Möglichkeit, die Inhalationen sowie deren Flussparameter mittels einer Sensorik zu erfassen. Zur Messung der Parameter benutzt man die Membran-Biegebalken-Technologie oder ein piezoresistives Element in Kombination mit einer Blende oder in Kombination mit dem Venturi-Messprinzip. Mit einer IPC-Logik und der Sensorik wird die Steuerung zur eine Regelung.

Diese Regelung erlaubt, über ein elektronisches Bewegungselement eine verstellbare Düse anzusteuern, die letztlich durch eine Widerstandsveränderung den Fluss im Inhalator konstant regelt.
- Zur Stromversorgung ist ein im Innern des Inhalators angeordneter Dynamo vorgesehen, der beim Öffnen der Schutzkappe **950** oder durch den Luftstrom im Inhalator während der Inhalation einen elektrischen Strom generiert, welcher gespeichert wird und dazu dient, die elektronischen Bauelemente zu versorgen.
- Die elektronischen Bauteile sind als steckbares, wiederverwendbares Steuermodul vom Inhalator lösbar, so dass ein Batteriebetrieb in Betracht kommt. Mittels eines integrierten Speicherbausteins werden die Inhalationsdaten gesammelt und für den Arzt bzw. Apotheker bereitgestellt. Damit wird eine exakte Kontrolle der Dosisabgabe möglich. Die Steckmodule sind an Basisgeräten für die weitere Verwendung neu aufladbar und/oder programmierbar, so dass nur der kontaminierte Teil des Inhalators auszusondern ist.
- Zur besseren Kontrolle des Inhalationsverlaufs wird mit Abschluss einer erfolgreichen bzw. misslungenen Inhalation ein mechanisch und/oder elektronisch erzeugtes akustisches und/oder optisches Signal abgegeben.
- Möglich ist es auch, die beiden zueinander komplementären Rasterabschnitte **515,621** einerseits am Schlitten **500** und andererseits am Gehäuse-Unterteil **100** oder am Gehäuse-Oberteil **150** anzuordnen. Damit nur während des Ausziehens der Schutzkappe **950** Vibrationen erfolgen - nicht aber beim Zurückschieben -, kann man einen der beiden Rasterabschnitte **515**,**621** jeweils beim Replazieren der Schutzkappe **950** ausser Funktion setzen, z.B. auf einem auch verschiebbaren Bauteil.
- Das jetzt im Trichterdeckel **680** innerhalb der Kammer **681** untergebrachte Trocknungspulver könnte man auch innerhalb der Trichterfassung **601** positionieren.
- Die Aussenkonturen des Inhalators sowie die innere Gewichtsverteilung bewirken bei Auflage auf eine im Prinzip waagerechte und formstabile Unterlage, dass sich der Inhalator stets mit nach unten weisendem Auslass **608** der Trichterfassung **601** ausrichtet.

## Patentansprüche

1. Mundstück (**900**) für einen Inhalator zur mehrfachen dosisweisen Abgabe eines pharmakologischen Trockenpulvers mit einem auf einem ersten, in der Gebrauchslage niedrigeren Niveau angeordneten Kanaleingang (**914**) sowie mit einem an einem Mundrohr (**920**) auf einem zweiten, in der Gebrauchslage höheren Niveau gelegenen Kanalausgang (**922**), wobei zwischen Kanaleingang (**914**) und Kanalausgang (**922**) eine Zerstäuberstrecke (**924**) zur Pulverdesagglomeration liegt, welche einen auf dem ersten Niveau angeordneten geraden oder gewundenen Kanalabschnitt, eine Schikane (**925**), sowie einen auf dem zweiten Niveau gelegenen Kanalabschnitt umfasst, der zum Kanalausgang (**922**) führt, **dadurch gekennzeichnet, dass** die Zerstäuberstrecke (**924**) einen im Volumen vergrösserten Kanalabschnitt (**928**) umfasst zur Reduktion der Pulverflussgeschwindigkeit und Abscheidung von gröberen, inhalativ unwirksamen Partikeln, und zum Umlenken des durchfliessenden Pulveraerosols von dem auf dem ersten Niveau gelegenen Kanalabschnitt zu dem auf dem zweiten Niveau gelegenen Kanalabschnitt, und dass der Kanalausgang **(922)** im oberen Bereich der Stirnseite **(921)** des Mundrohrs **(920)** gelegen ist und die Form eines Querschlitzes aufweist.

2. Mundstück (**900**) nach Anspruch 1, **dadurch gekennzeichnet, dass** es eine Steckverbindung (**911**) aufweist zur Anbringung am Gehäuse (**100,150**) eines Inhalators (**1**).

3. Mundstück (**900**) nach Anspruch 1 oder 2, **dadurch gekennzeichnet, dass** das Mundstück (**900**) mehrteilig und aufklappbar ausgebildet ist.

4. Mundstück (**900**) nach Anspruch 3, **dadurch gekennzeichnet, dass** die Teile des Mundstücks (**900**) über ein Filmscharnier (**923**) miteinander verbunden sind.

5. Mundstück (**900**) nach Anspruch 4, **dadurch gekennzeichnet, dass** das Mundstück (**900**) aus zwei zueinander symmetrischen Teilen besteht, und dass das Filmscharnier (**923**) an der äusseren Stirnseite (**921**) des Mundstücks (**900**) angeordnet ist.

## Claims

1. Mouthpiece (900) for an inhaler for multiple dosed administration of a pharmacological dry powder, having a channel inlet (914) being arranged on a first level that is lower in the use position, and having channel outlet (922) being arranged on a second level that is higher in the use position and being arranged at a mouth tube (920), wherein for the deagglomeration of powder there is arranged an atomiser path (924) between the channel inlet (914) and the channel outlet (922), which atomiser path comprises a straight or curved channel section arranged on the first level, a baffle (925), and a channel section being arranged on the second level and leading to the channel outlet (922), **charaterised in that** the atomiser path (924) comprises a channel section (928) with an enlarged volume for the reduction of the powder flow rate and for the separation of coarser particles ineffective for inhalation, and for deflecting the powder aerosol flowing through it from the channel section being arranged on the first level to the channel section being arranged on the second level, and wherein the channel outlet (922) is located in the upper portion of the end face (921) of the mouth tube (920) and has the form of a transverse slit.

2. Mouthpiece (900) according to claim 1, **characterised in that** it has a plug connection (911) for arrangement on the housing (100,150) of an inhaler (1).

3. Mouthpiece (900) according to claim 1 or 2, **characterised in that** the mouthpiece (900) is a multi-part, hinged mouthpiece.

4. Mouthpiece (900) according to claim 3, **characterised in that** the parts of the mouthpiece (900) are connected to one another via a film hinge (923).

5. Mouthpiece (900) according to claim 4, wherein the mouthpiece (900) consists of two include two halves symmetrical to one another, and that the film hinge (923) is arranged on the outer end face (921) of said mouthpiece (900).

## Revendications

1. Embout buccal (900) pour un inhalateur fournissant des doses multiples d'une poudre sèche pharmacologique avec une entrée de canal (914) disposée à un premier niveau, inférieur en position d'utilisation, ainsi qu'avec une sortie de canal (922) située à un deuxième niveau, supérieur en position d'utilisation, au niveau d'un tuyau buccal (920), une voie d'atomisation (924) se trouvant entre l'entrée de canal (914) et la sortie de canal (922) pour la désagglomération de la poudre, la voie comprenant un tronçon de canal droit ou sinueux, disposé au premier niveau, une chicane (925) ainsi qu'un tronçon de canal situé au deuxième niveau et menant à la sortie de canal (922), **caractérisé en ce que** la voie d'atomisation (924) comprend un tronçon de canal (928) d'un volume agrandi permettant de réduire la vitesse d'écoulement de la poudre et de déposer des particules plus grossières, sans effet pour l'inhalation, et de dévier l'aérosol de poudre de passage du tronçon de canal situé au premier niveau vers le tronçon de canal situé au deuxième niveau, et **en ce que** la sortie de canal (922) se trouve dans la partie supérieure de la face avant (921) du tuyau buccal (920) et présente la forme d'une fente transversale.

2. Embout buccal (900) selon la revendication 1, **caractérisé en ce qu'**il présente un connecteur (911) pour la mise en place sur le boîtier (100, 150) d'un inhalateur (1).

3. Embout buccal (900) selon la revendication 1 ou 2, **caractérisé en ce que** l'embout buccal (900) est réalisé en plusieurs pièces et de façon rabattable.

4. Embout buccal (900) selon la revendication 3, **caractérisé en ce que** les pièces de l'embout buccal (900) sont reliées ensemble par l'intermédiaire d'une charnière en forme de film (923).

5. Embout buccal (900) selon la revendication 4, **caractérisé en ce que** l'embout buccal (900) se compose de deux pièces symétriques l'une par rapport à l'autre, et **en ce que** la charnière en forme de film (923) est disposée sur la face avant extérieure (921) de l'embout buccal (900).
